(19) Europäisches Patentamt
European Patent Office
Office européen des brevets

(11) Numéro de publication: **0 251 938 B1**

# (12) FASCICULE DE BREVET EUROPEEN

(45) Date de publication de fascicule du brevet: **06.11.91**

(51) Int. Cl.[5]: **C07D 209/16**, C07D 413/06

(21) Numéro de dépôt: **87401563.9**

(22) Date de dépôt: **03.07.87**

## (54) Procédé de synthèse stéréospécifique de dérivés de l'indole.

(30) Priorité: **03.07.86 FR 8609680**

(43) Date de publication de la demande:
**07.01.88 Bulletin 88/01**

(45) Mention de la délivrance du brevet:
**06.11.91 Bulletin 91/45**

(84) Etats contractants désignés:
**AT BE CH DE ES FR GB GR IT LI LU NL SE**

(56) Documents cités:
**EP-A- 0 025 727**
**EP-A- 0 092 450**
**GB-A- 2 001 633**
**GB-A- 2 141 121**
**US-A- 4 234 595**

(73) Titulaire: **SANOFI**
**40, Avenue George V**
**F-75008 Paris(FR)**

(72) Inventeur: **Wagnon, Jean Le Hameau de la Rauze**
**Villa 34, Avenue du Pont Trinquet**
**F-34000 Montpellier(FR)**
Inventeur: **Plouzane, Claude**
**5, Allée des Terres Rouges**
**F-34680 Saint Georges D'Orques(FR)**
Inventeur: **Tonnerre, Bernard**
**Lotissement le Vallon, No. 3**
**F-34570 Vailhauques(FR)**
Inventeur: **Nisato, Dino**
**4, Impasse de l'Olivette**
**F-34680 Saint Georges D'Orques(FR)**

(74) Mandataire: **Gillard, Marie-Louise et al**
**Cabinet Beau de Loménie 55, Rue d'Amsterdam**
**F-75008 Paris(FR)**

EP 0 251 938 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet un procédé de synthèse asymétrique de dérivés de l'indole optiquement purs, répondant à la formule :

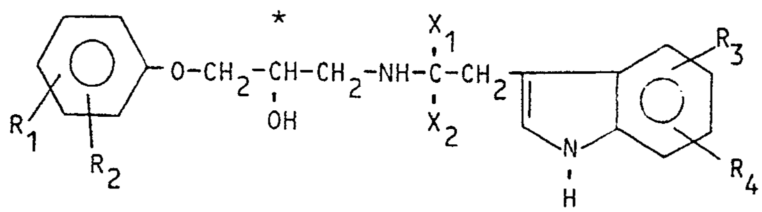

(I)

dans laquelle :

- l'atome C* a la configuration (R) ou (S) ;
- $X_1$ et $X_2$ désignent chacun indépendamment un atome d'hydrogène ou un alkyle droit ou ramifié comportant de 1 à 4 atomes de carbone ;
- $R_1$ et $R_2$ désignent chacun indépendamment un atome d'hydrogène, un atome d'halogène, un alkyle droit ou ramifié comportant de 1 à 4 atomes de carbone, un alcoxy droit ou ramifié comportant de 1 à 4 atomes de carbone, un formyle, ou $R_1$ et $R_2$ pris ensemble avec le noyau benzénique auquel ils sont liés forment un noyau bicyclique choisi parmi ceux de formule :

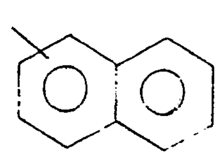 ; 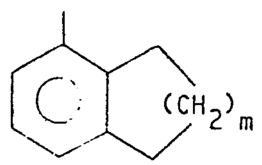 ; 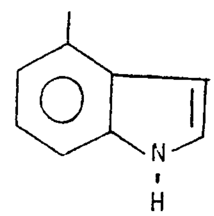

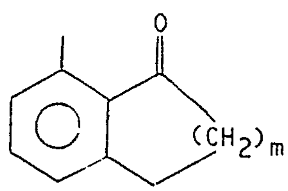 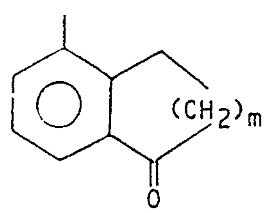

- $R_3$ et $R_4$ désignent chacun indépendamment un atome d'hydrogène, un atome d'halogène, un alkyle droit ou ramifié comportant de 1 à 4 atomes de carbone ou un alcoxy droit ou ramifié comportant de 1 à 4 atomes de carbone ;
- m représente un nombre entier compris entre 1 et 3.

Lorsque $X_1$ est différent de $X_2$, (I) peut avoir l'une des configurations suivantes (R,R), (R,S), (S,R) ou (S,S).

Le signe C* désigne un carbone asymétrique.

Les dérivés de l'indole (I) obtenus par le procédé selon l'invention sont des composés actifs sur le système cardiovasculaire. Ces composés sont utiles dans la préparation de médicaments destinés au traitement d'affections cardiovasculaires. Il est connu que l'activité pharmacologique de composés tels que les dérivés de formule (I) varie selon l'isomère étudié. Ainsi, pour le propanolol, les résultats sont reportés dans "Pharmacology of Antihypertension Drugs", A. SCRIABINE ; Raven Press, 1980, p. 197.

Les composés préparés par le procédé selon l'invention ont été testés par leurs effets alpha- et bêta-adrènergiques. Ainsi, on a constaté que le chlorhydrate de S(-) (diméthyl-1,1 (indolyl-3)-2 éthylamino)-3

(indolyl-4 oxy)-1 propanol-2 présente des effets alpha-, bêta-1 et bêta-2 adrénergiques supérieurs à ceux du chlorhydrate de R(+) (diméthyl-1,1 (indolyl-3)-2 éthylamino)-3 (indolyl-4 oxy)-1 propanol-2.

Le procédé de synthèse selon l'invention est stéréospécifique sur le carbone portant la fonction alcool.

Le brevet EP 25 727, le brevet FR 2 523 964 et le brevet US 4 314 943 décrivent un procédé de synthèse de mélange de stéréoisomères de certains composés (I') par condensation d'un époxyde sur une amine selon le schéma réactionnel ci-aprés :

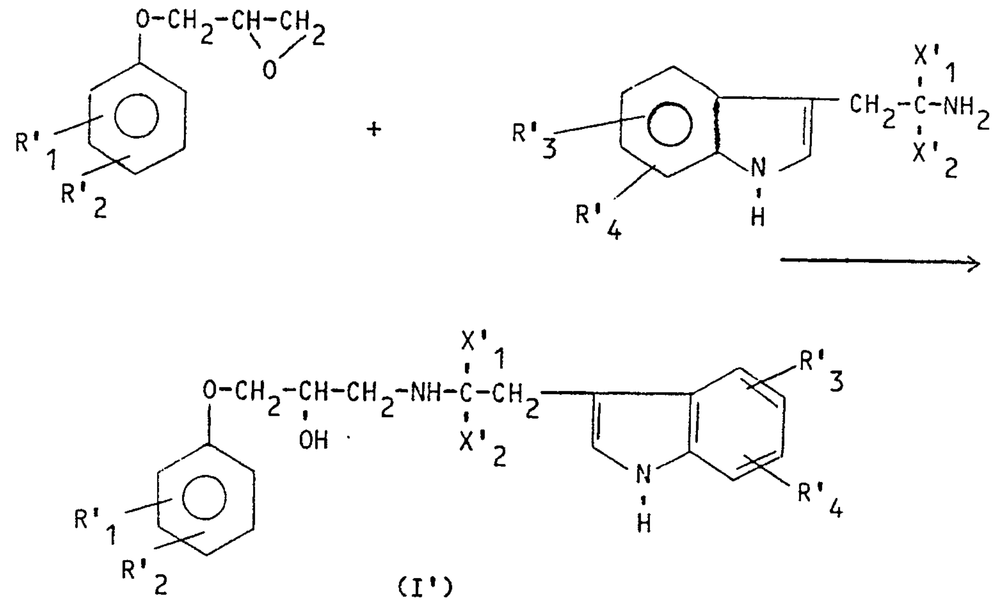

(I')

Par ce procédé de synthèse, les composés (I') sont obtenus sous forme d'un mélange de 2 isomères optiques lorsque $X'_1 = X'_2$ ou d'un mélange de 4 stéréoisomères lorsque $X'_1$ et $X'_2$ ont des valeurs différentes. La séparation de chacun des isomères optiques nécessite l'application de méthodes physiques ou chimiques appropriées qui allongent le procédé et diminuent le rendement en produit optiquement pur.

Dans la demande GB-A-2 001 633 et le brevet US-A-4 234 595, on décrit des dérivés indoliques et leur préparation. Il est mentionné la possibilité de les obtenir de façon stéréospécifique. Toutefois, aucune indication n'est précisément donnée pour permettre la mise en oeuvre du procédé devant assurer ladite stéréospécificité.

Ledit procédé consisterait principalement en l'action d'un hydroxyaryle (ArOH) sur un composé de formule

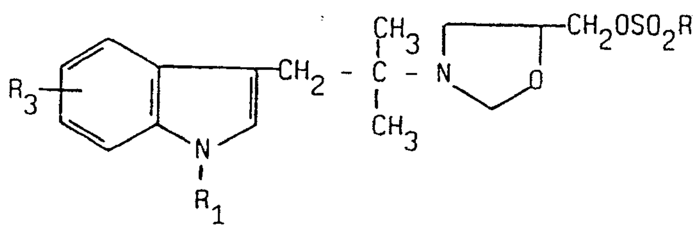

obtenu avec du glycéraldéhyde optiquement pur.

Selon la présente invention, on propose de préparer les dérivés de l'indole de formule (I) par un procédé de synthèse stéréospécifique sur le carbone portant la fonction alcool. Selon ledit procédé, on fait intervenir, sous forme optiquement pure le tosyloxy-3-0-isopropylidène-1,2 propanediol-1,2.

Dans la description et dans les exemples qui vont suivre, on utilisera les abréviations suivantes :

| | |
|---|---|
| Boc : | tert-butyloxycarbonyle |
| $(Boc)_2O$ : | anhydride de l'acide tert-butyloxycarbonique |
| THF : | tétrahydrofuranne |
| DMF : | diméthylformamide |

$T_S$ : tosyle
CCM : chromatographie sur couche mince.

Le procédé selon l'invention consiste à utiliser le tosyloxy-3 0-isopropylidène-1,2 propanediol-1,2 (II) sous une forme optiquement pure pour introduire le carbone asymétrique C* du composé (I). Le composé (II) sous forme (R) est commercial, le composé antipode (S) s'obtient par des méthodes connues. En condensant le composé (II) avec une amine primaire (III) dérivée de la triptamine, convenablement choisie, on obtient le composé (IV) dans lequel la stéréochimie de C* est conservée : en condensant le (R) tosyloxy-3 O-isopropylidène-1,2 propanediol-1,2 avec (III), le C* de (IV) a la configuration (S) et,en condensant le (S) tosyloxy-3 O-isopropylidène-1,2 propanediol-1,2 avec (III), le C* de (IV) a la configuration (R). (Règles de priorité, R.S. CAHN, C. INGOLD, V. PRELOG, Angew. Chem. Int. Ed. Eng. (1966), 5, 385).

Le schéma réactionnel est le suivant :

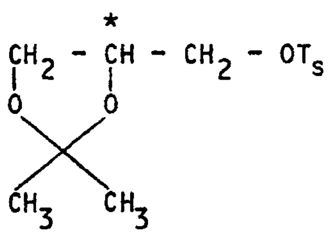

+

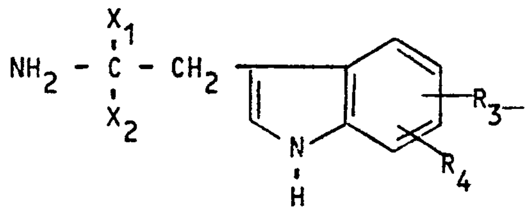

(II) (III)

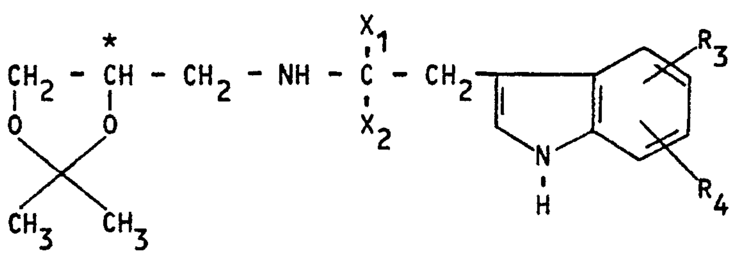

(IV)

Cette réaction est effectuée par chauffage dans un solvant inerte. Une partie de l'amine (III) se transforme dans la réaction en sel de l'acide p-toluènesulfonique de cette amine ; si la réaction a été réalisée en présence d'un excès de l'amine (III), on régénère avantageusement (III) en excès en traitant le milieu réactionnel par de la soude.

Lorsque les substituants $X_1$ et $X_2$ sont différents, l'amine (III) présente un centre d'asymétrie. Dans ce cas, si la formation de (IV) est effectuée à partir d'un isomère optiquement pur de (III), la chiralité du carbone portant $X_1$ et $X_2$ est conservée et le composé (IV) est optiquement pur. On peut également préparer (IV) à partir d'une amine (III) sous forme racémique et séparer les épimères dans une étape ultérieure.

A partir de (IV) on effectue successivement et dans un ordre quelconque l'hydrolyse en milieu acide du cycle dioxolanne et la substitution de la fonction amine par un groupement oxycarbonyle ROCO dans lequel R représente un alkyle droit ou ramifié comportant de 1 à 4 atomes de carbone ou un benzyle pour former le composé (VI).

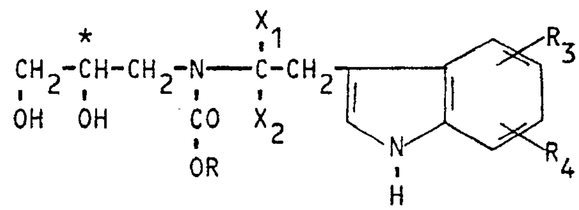

(VI)

Dans cette suite de réactions, la chiralité de C* est conservée. Le cas échéant, la chiralité de C* portant $X_1$ et $X_2$ est également conservée.

Ainsi, selon un mode de réalisation, le cycle dioxolanne du composé (IV) est ouvert par hydrolyse en milieu acide pour donner le diol-1,2 correspondant (V) puis la fonction amine de (V) est substituée par un groupement oxycarbonyle ROCO dans lequel R représente un alkyle droit ou ramifié comportant de 1 à 4 atomes de carbone ou un benzyle, tel que le tert-butyloxy-carbonyle (Boc) ou le méthyloxycarbonyle pour obtenir (VI).

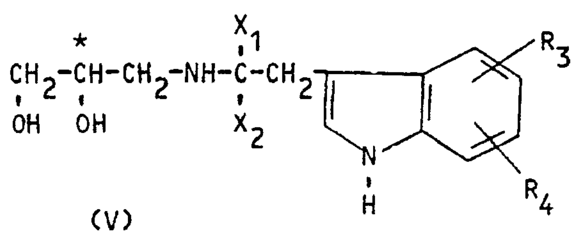

(V)

Selon un autre mode de réalisation, on effectue d'abord la substitution de l'amine du composé (IV) par un groupement oxycarbonyle ROCO pour former le composé (IV'), puis on réalise l'hydrolyse en milieu acide du cycle dioxolanne de (IV') pour obtenir le composé (VI).

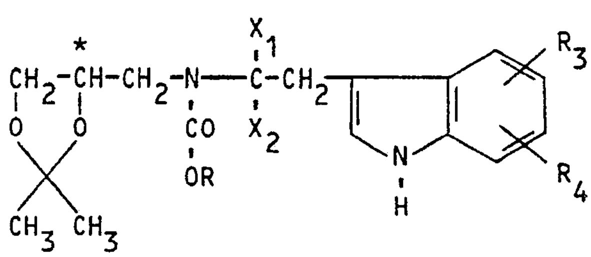

(IV')

Par chauffage en milieu basique du composé (VI) dans un mélange eau-alcool ou dans un solvant non aqueux, tel que le DMF, on forme l'oxazolidinone (VII) :

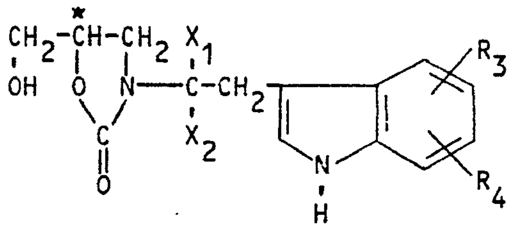

(VII)

La réaction peut être effectuée dans un mélange eau-alcool ou dans un solvant non aqueux tel que le DMF. Lorsque la réaction est effectuée dans un solvant contenant de l'eau, le chauffage doit être contrôlé pour éviter l'ouverture du cycle oxazolidine formé.

Eventuellement, on peut effectuer une protection sélective temporaire de l'azote indolique de l'un des composés (IV') ou (VII) par un groupement, tel que le tosyle ou le mésyle,par action du chlorure de tosyle ou de mésyle dans le DMF à température ordinaire.

Après estérification de la fonction alcool de (VII) par du chlorure de tosyle ou du chlorure de mésyle dans la pyridine à température ordinaire ou de préférence à une température inférieure ou égale à 0 ° C, on fait agir un phénol de formule (VIII), en présence d'un agent alcalin tel que la soude, la potasse, un alcoolate de sodium ou de potassium ou l'hydrure de sodium, dans un solvant approprié, ledit phénol répond à la formule :

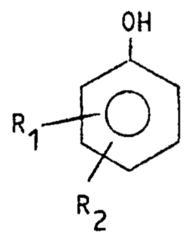

(VIII)

dans laquelle $R_1$ et $R_2$ ont les significations indiquées plus haut. On obtient le composé (IX) dans lequel la chiralité de C* est conservée ainsi que, le cas échéant, la chiralité de C* portant $X_1$ et $X_2$.

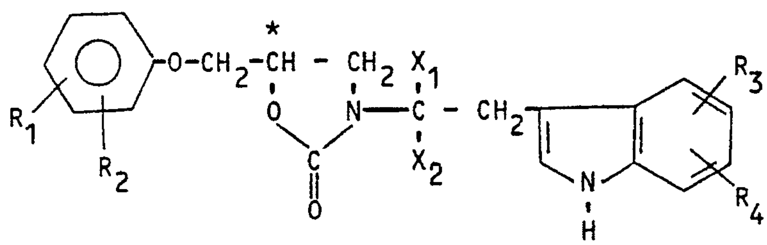

(IX)

L'ouverture de l'oxazolidinone (IX) pour obtenir les dérivés de l'indole (I) est réalisée en milieu basique par chauffage au reflux en présence d'eau dans un solvant convenable tel qu'un alcool, un glycol, un éther de glycol ou un solvant dipolaire comme le disulfolane. Lorsque le phénol (VIII) porte une ou deux fonctions formyles susceptibles de réagir aux agents alcalins, celles-ci peuvent être protégées sous forme de dioxolanne avant la formation de l'oxazolidinone (IX). La fonction formyle est ensuite régénérée par des méthodes connues de la chimie organique.

Lorsque $X_1$ est différent de $X_2$, si l'amine (III) est optiquement pure, les composés intermédiaires (IV), (IV') ou (V), (VI), (VII), (IX) et le composé (I) sont optiquement purs. Si l'amine (III) est sous forme racémique, le composé (IV) est un mélange d'épimères. La résolution peut être effectuée après toute étape du procédé, c'est-à-dire à partir de l'un des composés (IV), (IV'), (V), (VI), (VII) ou (IX) ou à la dernière étape sur le composé (I).

Le cas échéant, on prépare ensuite un sel de (I) pharmaceutiquement acceptable avec un acide minéral

ou organique tel que l'acide chlorhydrique, l'acide fumarique ou l'acide maléique.

A partir de composés (I) préparés selon l'invention, on peut obtenir d'autres dérivés de l'indole en effectuant des réactions appropriées sur (I). Par exemple, lorsque $R_1$ = CHO et $R_2$ = H, on peut préparer l'oxime correspondante de (I), puis le dérivé cyano et éventuellement le dérivé amide correspondant de (I).

Le procédé selon l'invention est également applicable à la préparation de dérivés de l'indole semblables à (I) portant divers substituants sur le noyau benzénique et sur le noyau indolique. Cependant, si le noyau benzénique porte une fonction susceptible de réagir avec les agents alcalins, cette fonction devra être protégée avant la formation de l'oxazolidinone semblable à (IX).

Dans le procédé selon l'invention, les intermédiaires (VII) et (IX) optiquement actifs sont nouveaux.

Ainsi, la présente invention a également pour objet les dérivés de l'hydroxyméthyl-5 (indolyl-3)-2 éthyl-3 oxazolidin-1,3 one-2 de formule :

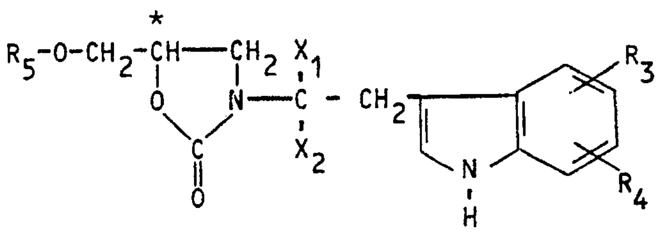

(X)

dans laquelle :

- l'atome C* a la configuration (R) ou (S)
- $R_5$ représente un atome d'hydrogène ou un groupe :

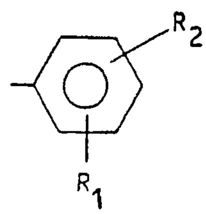

- et $X_1$, $X_2$, $R_1$, $R_2$, $R_3$, $R_4$ ont les valeurs indiquées ci-dessus pour les composés de formule (I).

Lorsque $X_1$ est différent de $X_2$, le composé (X) a l'une des configurations suivantes : (R,R), (R,S), (S,S), (S,R) ou le mélange d'épimères (R,R) (R,S) ou (S,R) (S,S).

Un mode de réalisation préférentiel du procédé selon l'invention consiste à condenser le (R) tosyloxy-3 0-isopropylidène-1,2 propanediol-1,2 sur la d,l-alpha méthyltryptamine (III ; $X_1$ = $CH_3$, $X_2$ = $R_3$ = $R_4$ = H) pour préparer la (2S) hydroxyméthyl-5 ((indolyl-3)-2 méthyl-1 éthyl)-3 oxazolidin-1,3 one-2 (VII) sur laquelle on fait réagir l'orthocrésol (VIII ; $R_1$ = ortho-$CH_3$, $R_2$ = H>. On obtient ainti la (2S) [(indolyl-3)-2 méthyl-1 éthyl]-3 (méthyl-2 phénoxy-méthyl)-5 oxazolidin-1,3 one-2 (IX) dont on sépare les 2 épimères (2S, 1'R) et (2S, 1'S). On prépare enfin le fumarate neutre de l'((indolyl-3)-2 méthyl-1 éthylamino)-1 (méthyl-2) phénoxy-3 propanol-2 (I) sous ses 2 formes épimères (2S, 1'R) et (2S, 1'S).

Un autre mode de réalisation préférentiel du procédé selon l'invention consiste à condenser le (R) tosyloxy-3 0-isopropylidène-1,2propanediol-1,2 sur l'alpha alpha-diméthyl tryptamine (III ; $X_1$ = $X_2$ = $CH_3$, $R_3$ = $R_4$ = H) pour préparer la (S) diméthyl-1,1 (indolyl-3)-2 éthyl)-3 hydroxyméthyl-5 oxazolidin-1,3 one-2 (VII) sur laquelle on fait réagir l'hydroxy-4 indole (VIII) sous la forme de N-tosyl hydroxy-4 indole. On obtient ainsi la (S) [diméthyl-1,1 (indolyl-3)-2 éthyl]-3 indolyl-4 oxyméthyl-5 oxazolidin-1,3 one-2 (IX). On prépare enfin le (S) [diméthyl-1,1 (indolyl-3)-2 éthytamino]-3 (indolyl-4) oxy-1 propanol-2.

Les exemples qui vont suivre illustrent l'invention sans toutefois la limiter.

Le pouvoir rotatoire (alpha D), exprimé en degré, est mesuré à 25° C ; on indique entre parenthèses la concentration du produit (c) et le solvant utilisé. Les points de fusion (Fc) sont mesurés au tube capillaire.

Le nom chimique des isomères optiques purs dont un seul centre d'asymétrie a une configuration connue ne précise que la configuration du seul centre défini.

Exemple 1

Fumarate neutre de (2S) [(indolyl-3)-2 méthyl-1 éthylamino]-1 (méthyl-2 phénoxy)-3 propanol-2.

1) - (2S) [(indolyl-3)-2 méthyl-1 éthylamino]-3 0-isopropylidène-1,2 propanediol-1,2.

On chauffe 60 g de (R) (-)tosyloxy-3 0-isopropylidène-1,2 propanediol-1,2 et 72 g de d,l-alpha méthyl tryptamine (2 équivalents) dans 300 ml de benzène pendant 72 heures. Après refroidissement, on filtre ; le solide récupéré est lavé avec du benzène, les phases organiques sont réunies et traitées avec du carbonate de sodium aqueux. On sèche sur sulfate de sodium puis le solvant est évaporé sous pression réduite et le résidu huileux est chromatographié sur silice en éluant avec un mélange acétate d'éthyle-méthanol (7/3 ; v/v). On obtient 57 g de produit sous forme huileuse.
Rendement 94 % ;
alpha D = + 9,5 (c = 1,85 ; chloroforme) ;
$C_{17}H_{24}N_2O_2$ :
Analyse : Calculé : C = 70,80 H = 8,39 N = 9,71
Trouvé : C = 70,69 H = 8,60 N = 9,67

Le solide recueilli par filtration est mis en suspension dans l'acétate d'éthyle, agité avec de la soude aqueuse. On récupère ainsi 30 g de d,l-alpha méthyl triptamine en excès.
Rendement 83 % ;
alpha D = 0.

2) - (2S) [(indolyl-3)-2 méthyl-1 éthylamino]-3 propanediol-1,2.

Le composé obtenu à l'étape précédente (50 g) est chauffé pendant 6 heures dans 600 ml d'une solution aqueuse d'acide acétique à 20 %. Après refroidissement, on laisse une nuit à température ambiante, une partie du solvant est évaporée à la trompe à eau jusqu'à un volume d'environ 150 ml. On extrait à l'éther puis on ajoute une solution aqueuse diluée de soude et on extrait la phase aqueuse 3 fois par de l'acétate d'éthyle. Après plusieurs lavages et séchages sur sulfate de sodium, on évapore le solvant et l'on obtient une huile brune.
Poids : 42 g ;
Rendement : 95 % ;
alpha D = -8,5 (c = 1,85 ; chloroforme) ;
$C_{14}H_{20}N_2O_2$:
Analyse : Calculé : C = 67, 71 H = 8,12 N = 11,28
Trouvé : C = 67,62 H = 8,18 N = 10,43

3) - (2S) [N-Boc (indolyl-3)-2 méthyl-1 éthylamino]-3 propanediol-1,2.

On porte à reflux pendant 7 heures une solution de 43 g de l'aminodiol obtenu à l'étape précédente et 32,5 g de $(Boc)_2O$ dans 200 ml de THF. Après une nuit à température ambiante, le solvant est évaporé sous pression réduite et le résidu est purifié par chromatographie sur silice. Le composé attendu est élué sous forme d'une huile par un mélange éther-dichlorométhane (1/1 ; v/v).
Poids : 41 g ;
Rendement : 68 % ;
alpha D = -7,6 (c = 1,1 ; méthanol) ;
$C_{19}H_{28}N_2O_2$:
Analyse : Calculé : C = 65,49 H = 8,04 N = 8,04
Trouvé : C = 64,74 H = 7,94 N = 7,84.

4) - (2S) Hydroxyméthyl-5 [(indolyl-3)-2 méthyl-1 éthyl]-3 oxazolidin-1,3 one-2.

Le composé obtenu à l'étape précédente (27 g) est dissous dans 270 ml d'éthanol puis on ajoute de l'eau et de la soude pour amener la solution à pH 13,5. Le mélange est chauffé à 64° C pendant 72 heures. Le mélange est ensuite concentré par évaporation d'une partie de l'éthanol à la pompe à eau, puis l'oxazolidinone est extraite par le dichlorométhane (4 fois 100 ml). Les phases organique sont rassemblées, séchées sur sulfate de sodium, le solvant est évaporé sous vide puis le résidu est purifié par chromatographie sur silice; en éluant par l'acétate d'éthyle pur, on obtient le composé attendu sous forme d'une huile.
Poids : 17 g ;

Rendement : 81 % ;
alpha D = + 22,7 (c = 1 ; méthanol) ;
$C_{15}H_{18}N_2O_2$:
Analyse : Calculé : C = 65,67 H = 6,56 N = 10,20
Trouvé : C = 65,03 H = 6,49 N = 9,87

5) - (2S) [(Indolyl-3)-2 méthyl-1 éthyl]-3 (méthyl-2 phénoxyméthyl)-5 oxazolidin-1,3 one-2.

Le composé obtenu à l'étape précédente (17 g) est dissous dans 75 ml de pyridine puis refroidi à -5° C. On ajoute alors en 30 minutes, par petites fractions, 12 g de chlorure de tosyle et l'on maintient à 0° C pendant 72 heures. On verse sur de l'eau glacée, extrait 4 fois au chlorure de méthylène, lave avec une solution d'acide chlorhydrique 2N jusqu'à pH acide, sèche et concentre à l'évaporateur rotatif à 30° C.

On obtient une huile (26 g). La présence de 2 isomères est visible en CCM sur silice (éluant : acétate d'éthyle).

On dissout 1,45 g de sodium dans 90 ml de méthoxyéthanol, on ajoute 6,8 g d'orthocrésol puis on chauffe à 120° C pendant 20 minutes. Le chauffage étant arrêté, on ajoute goutte à goutte en 30 minutes le produit obtenu ci-dessus (26 g), on chauffe à 120° C pendant 3 heures puis laisse revenir à température ambiante une nuit. On verse sur de l'eau glacée puis on extrait 5 fois au chlorure de méthylène, on lave la phase organique avec une solution de soude 1N, sèche et concentre. On obtient une huile que l'on chromatographie sur une colonne de silice préparée avec un mélange acétate d'éthyle-pentane (80/20 ; v/v). On élue avec l'acétate d'éthyle pur pour obtenir un mélange des 2 isomères que l'on va ensuite séparer. On filtre les cristaux qui apparaissent dans différentes fractions pour obtenir 5 g du mélange riche en isomère le moins polaire et on conserve les eaux mères. On effectue une cristallisation fractionnée dans l'acétate d'éthyle du mélange riche en isomère le moins polaire pour obtenir des cristaux purs à 95 % en RMN.
Poids : 3 g ;
alpha D = + 83 (c = 1 ; méthanol).

Ce composé est le produit attendu dans la configuration (2S,1'R).

On concentre les eaux mères pour obtenir 7,9g du mélange riche en isomère le plus polaire, ce mélange est chromatographié sur silice en éluant par le mélange pentane-acétate d'éthyle (50/50 ; v/v).
On obtient 2,8 g d'huile ;
alpha D = + 27,2 (c = 1 ; méthanol) ;
Ce composé est le produit attendu dans la configuration (2S, 1'R).

6) - Fumarate neutre de (2S, 1'S) [(indolyl-3)-2 méthyl-1 éthylamino]-1 (méthyl-2 phénoxy)-3 propanol-2.
SR 41 410 A.

On porte au reflux pendant 72 heures une suspension de 3,45 g de l'isomère le moins polaire isolé à l'étape précédente dans 50 ml d'éthanol et 45 ml de soude 4N. On évapore le solvant, reprend par 50 ml d'eau, extrait 3 fois au chlorure de méthylène, sèche et concentre. L'huile obtenue (3,3 g) placée dans 30 ml d'acétate d'éthyle chaud est traitée par un équivalent d'acide fumarique dissous dans le minimum d'éthanol bouillant ; les cristaux obtenus sont recristallisés dans le mélange éthanol/eau (60/40 ; v/v).
Poids : 1,4 g ;
Fc = 162-163° C.

Les eaux mères de recristallisation sont concentrées, traitées par la soude puis l'acide fumarique et recristallisées dans les mêmes conditions.
Poids : 700 mg ;
Fc = 162-163° C ;
Poids total du composé attendu : 2,1 g ;
Rendement : 56 % ;
alpha D = 0 (c = 1 ; éthanol/eau : 60/40) ;
Pouvoir rotatoire mesuré en lumière polarisée
à 400 nm : + 7°
(c = 1 ; éthanol/eau : 60/40 ; v/v) ;
$C_{23}H_{28}N_2O_4$ :
Analyse : Calculé : C = 69,68 H = 7,12 N = 7,06
Trouvé : C = 69,27 H = 7,00 N = 6,97.

7) - Fumarate neutre de (2S,1'R) [(indolyl-3)-2 méthyl-1 éthylamino]-1 (méthyl-2 phénoxy)-3 propanol-2. SR 41 475 A.

On porte au reflux pendant 72 heures une suspension de 1,8 g du composé le plus polaire (enrichi à 90 %), isolé à l'étape 5, dans 50 ml d'éthanol et 45 ml de soude 4N. On évapore le solvant, reprend par 50 ml d'eau, extrait au chlorure de méthylène, sèche et concentre. On chromatographie sur colonne de silice en éluant par un mélange acétate d'éthyle/méthanol (90/10 ; v/v) pour obtenir 1,5 g de produit que l'on traite par un équivalent d'acide fumarique dans le mélange acétate d'éthyle/méthanol (90/10 ; v/v). Les cristaux obtenus sont recristallisés dans le mélange éthanol/eau (60/40 ; v/v), on obtient 1,25 g du composé attendu.
Rendement : 64 % ;
Fc = 166-167° C ;
alpha D = - 29,2 (c = 0,5 ; éthanol/eau ; 60/40 ; v/v) ;
$C_{23}H_{28}N_2O_4$ :
Analyse : Calculé : C = 69,68 H = 7,12 N = 7,06
Trouvé : C = 68,59 H = 7,10 N = 6,77.

Les configurations absolues des produits préparés à l'étape 5 et des composés SR 41 410 A, obtenu à l'étape 6,et SR 41 475 A, obtenu à l'étape 7, ont été déterminées par rapport à la configuration absolue de leurs énantiomères : le couple d'épimères ayant un alcool de configuration inverse (R) et préparés à partir de chacune des 2 alpha méthyl triptamines résolues de configuration absolue connue.

Ainsi, on a préparé les fumarates neutres (2R, 1'S) et (2R, 1'R) et l'[(indolyl-3)-2 méthyl-1 éthylamino]-1 (méthyl-2 phénoxy)-3 propanol-2 selon le mode opératoire décrit ci-dessous.

A) - Résolution de l'alpha méthyl tryptamine.

a) (S) (+) alpha méthyl tryptamine.

A une solution de d,l-alpha méthyl tryptamine (40 g) dans 300 ml d'isopropanol, on ajoute 55,2 g d'acide d-camphor sulfonique dissous dans le minimum d'isopropanol. Après une nuit à température ambiante, le sel est filtré, on obtient 76 g qui sont recristallisés 4 fois d'un mélange méthanol-isopropanol (1/4 ; v/v). Le sel obtenu est dissous dans l'eau puis on ajoute 10 g de soude en pastilles, la base libre est extraite par du dichlorométhane, la phase organique est séchée sur sulfate de sodium et le solvant est évaporé. Le solide obtenu est cristallisé par dissolution dans du dichlorométhane puis addition d'éther isopropylique.
On obtient 8 g de S(+) alpha méthyltryptamine ;
Rendement : 20 % ;
Fc = 122-123,5° C ;
alpha D = + 43,2 (c = 1,05 ; éthanol absolu) ;
$C_{11}H_{14}N_2$ :
Analyse : Calculé : C = 76,26 H = 7,57 N = 16,17
Trouvé : C = 75,56 H = 8,11 N = 15,44

b) (R) (-) alpha méthyl tryptamine.

On récupère 25,6 g de d,l-alpha méthyl tryptamine enrichi en épimère (R) (-) à partir des eaux mères obtenues à l'étape précédente après le traitement par la soude. Ce composé est salifié par 58 g d'acide O,O'-bis p-toluoyltartrique. Le sel obtenu est recristallisé 3 fois dans 900 ml de mélange éthanol-eau (2/1 ; v/v). 31 g de sel sont ainsi isolés puis recristallisés par dissolution dans du dichlorométhane puis addition d'éther isopropylique.
On obtient 7,7 g ;
Fc = 122-123,5° C ;
alpha D = -43 (c = 1,0 ; éthanol absolu) ;
$C_{11}H_{14}N_2$ :
Analyse : Calculé : C = 76,26 H = 7,57 N = 16,17
Trouvé : C = 75,50 H = 8,08 N = 15,97.

B) - (R) (+) (méthyl-2 phénoxyméthyl)-3 propanediol-1,2.

On prépare une solution de sel de sodium d'ortho crésol en portant à reflux 7 ml de méthoxyéthanol anhydre, 460 mg de sodium et 2,16 g d'ortho crésol. A cette solution, on ajoute en 15 minutes 5,72 g de (R) (-) tosyloxy-3 O-isopropylidène-1,2 propanediol-1,2 dissous dans 10 ml de méthoxyéthanol et on porte 3 heures à reflux. Après refroidissement, on ajoute de l'eau et la phase organique est extraite 3 fois avec de l'hexane. La phase organique est lavée 2 fois avec une solution diluée de soude puis séchée sur sulfate de sodium et le solvant est évaporé. On obtient 3,3 g d'une huile jaune qui est dissoute dans 16 ml d'acide acétique, on ajoute 4 ml d'eau puis on chauffe à 70° C pendant 90 minutes. Après une nuit à température ambiante, on ajoute de l'eau puis on neutralise lentement par addition de carbonate acide de sodium. La phase organique est extraite par de l'éther éthylique. Après lavage puis séchage sur sulfate de sodium, le solvant est évaporé et le résidu est cristallisé du mélange dichlorométhane-éther isopropylique.
On obtient 2,2 g ;
Fc = 88° C ; littérature 89-90° C ;
Pouvoir rotatoire mesuré en lumière polarisée à 400 nm = +12,8
(c = 5,07 ; chloroforme).

C) - (S) (+) (méthyl-2 phénoxyméthyl)-3 tosyloxy-1 propanol-2.

On refroidit à -13° C une solution de 5,5 g du composé obtenu à l'étape précédente dans 25 ml de pyridine. On ajoute en 30 minutes 5,75 g de chlorure de tosyle fraîchement recristallisé dans l'hexane. Le mélange est gardé 8 jours à 0° C en agitant de temps en temps. On ajoute ensuite à 0° C 100 ml d'acétate d'éthyle puis 150 ml d'acide sulfurique à 20 %. La phase organique est décantée et le résidu est extrait 3 fois à l'acétate d'éthyle. Les extraits sont rassemblés, lavés jusqu'à la neutralité par une solution aqueuse de carbonate acide de sodium puis de l'eau saline et on sèche sur sulfate de sodium. Par évaporation sous vide à 50° C, on obtient 8,5 g d'une huile que l'on purifie par chromatographie sur colonne de silice en éluant par de l'éther.
On obtient 7,85 g ;
Rendement : 78 % ;
alpha D à 26° C = +12,8 (c = 8,39 ; chloroforme).

D) - Fumarate neutre de (2R, 1'R) [(indolyl-3)-2 méthyl-1 éthylamino]-1 (méthyl-2 phénoxy)-3 propanol-2.
SR 41 624 A.

On mélange dans 250 ml de benzène 3,4 g du composé obtenu à l'étape précédente et 3,5 g de (R) (-) alpha méthyl tryptamine préparé à la première étape et l'on chauffe à reflux pendant 18 heures. Le solvant est évaporé sous vide et l'on ajoute au résidu 250 ml de dichlorométhane puis une solution de soude concentrée. La phase organique est décantée, séchée sur sulfate de sodium puis le solvant est évaporé. Le résidu est purifié par chromatographie sur colonne de silice en éluant par un mélange acétate d'éthyle-méthanol (9/10 ; v/v). On obtient 2 g de produit sous forme d'une huile. Le sel est préparé dans 40 ml du mélange éther-acétate d'éthyle (3/1 ; v/v) contenant 0,68 g d'acide fumarique.
On obtient 1,35 g ;
Rendement : 29 % ;
Fc = 162-163° C ;
alpha D = 0 (c = 0,5 ; éthanol/eau : 6/4, v/v) ;
Pouvoir rotatoire mesuré en lumière polarisée à 400 nm = -8,2 (c = 0,5 ; éthanol/eau : 6/4, v/v) ;
$C_{23}H_{28}N_2O_4$ :
Analyse : Calculé : C = 69,68 H = 7,12 N = 7,06
Trouvé : C = 69,56 H = 7,59 N = 6,79.

E) - Fumarate neutre de (2R,1'S) [(indolyl-3)-2 méthyl-1 éthylamino]-1 (méthyl-2 phénoxy)-3 propanol-2.
SR 41 583 A.

Ce composé est obtenu à partir de 5,2 g de (S) (+) alpha méthyl tryptamine et 5 g de (S) (+) (méthyl-2 phénoxyméthyl)-3 tosyloxy-1 propanol-2 en utilisant la méthode précédemment décrite. La formation du sel d'acide fumarique est effectuée dans 45 ml de mélange acétate d'éthyle-méthanol (2/1 ; v/v) puis cristallisation dans l'éthanol.
On obtient 1,75 g du composé attendu ;
Rendement 30 % ;
Fc 169-170° C ;

alpha D = +31,4 (c = 1 ; éthanol/eau : 6/4, v/v) ;
Pouvoir rotatoire mesuré en lumière polarisée à 400 nm = +81,4 (c = 1 ; éthanol/eau : 6/4, v/v) ;
$C_{23}H_{28}N_2O_4$ :
Analyse : Calculé : C = 69,68 H = 7,12 N = 7,06
Trouvé : C = 69,89 H = 7,06 H = 6,97.

Exemple 2

(S) (-) [Diméthyl-1,1 (indolyl-3)-2 éthylamino]-3 (indolyl-4 oxy)-1 propanol-2 ; (S) (-) CM 40441.

1) - (S) (+) [Diméthyl-1,1 (indolyl-3)-2 éthylamino]-3 0-isopropylidène-1,2 propanediol-1,2.

On chauffe au reflux du toluène (250 ml) pendant 66 heures un mélange de 15,5 g de (R) (-) tosyloxy-3 0-isopropylidène-1,2 propanediol-1,2 et 20,1 g d'alpha,alpha-diméthyl tryptamine. On refroidit à 70° C, ajoute 100 ml de soude 3N et agite énergiquement pendant 5 minutes puis on laisse revenir à température ambiante. Le solide qui précipite est filtré. En recristallisant 2 fois par dissolution dans le chloroforme puis addition du minimum d'éther isopropylique, on récupère 7,5 g d'amine en excès (74 % de l'excès).

Les jus de la première filtration sont lavés par de la soude diluée; après séchage sur sulfate de magnésium, on évapore le toluène et le résidu est chromatographié sur gel de silice. Le produit attendu est élué par de l'acétate d'éthyle puis le mélange acétate d'éthyle-méthanol (95/5 ; v/v).
On obtient un solide blanc : 11,35 g ;
Rendement : 69,5 % ;
alpha D = +2,18 (c = 1,92 ; chloroforme).

Un échantillon est recristallisé par dissolution dans l'éther isopropylique et addition du minimum d'éther isopropylique.
Fc = 100-102° C ;
alpha D = +2,21 (c = 3,16 ; chloroforme).
$C_{18}H_{26}N_2O_2$ :
Analyse : Calculé : C = 71,49 H = 8,67 N = 9,26
Trouvé : C = 71,29 H = 8,65 N = 9,13.

2) - (S) [Diméthyl-1,1 (indolyl-3)-2 éthylamino]-3 propanediol-1,2.

7,2 g du composé obtenu à l'étape précédente sont chauffés à 80° C pendant 7 heures dans 130 ml d'eau contenant 20 ml d'acide acétique. On refroidit dans la glace puis alcalinise par de la soude concentrée, extrait 3 fois par l'acétate d'éthyle, lave par de l'eau, de l'eau saline, sèche sur sulfate de magnésium et évapore. Le résidu obtenu (6,8 g) est cristallisé une fois par dissolution dans le dichlorométhane et addition du minimum d'éther isopropylique.
On obtient 4,2 g du produit attendu.
Rendement 67 % ;
Fc = 77-79° C.

3) - (S) (N-Boc diméthyl-1,1 (indolyl-3)-2 éthylamino)-3 propanediol-1,2.

Le composé obtenu à l'étape précédente (3,5 g) est chauffé dans 120 ml de THF avec un équivalent de $(Boc)_2O$ (2,9 g) pendant 3 jours. On évapore sous vide et chromatographie sur gel de silice en éluant par le mélange hexane-acétate d'éthyle.
On obtient 1,4 g du produit attendu.
Rendement 29 % ;
Spectre d'absorption infrarouge dans le dichlorométhane : lambda (nm) = 3560, 3480, 1696, 1680.
$C_{20}H_{30}N_2O_4$ :
Analyse : Calculé : C = 66,27 H = 8,34 N = 7,73
Trouvé : C = 65,85 H = 8,38 N = 7,29.

4) - (S) (+) [Diméthyl-1,1 (indolyl-3)-2 éthyl]-3 hydroxyméthyl-5 oxazolidin-1,3 one-2.

On dissout le composé obtenu à l'étape précédente (1,7 g) dans 30 ml d'éthanol puis on ajoute 30 ml d'eau amenée à pH 13,5 par addition de soude et on chauffe à 60° C pendant 36 heures. On neutralise par

de l'acide chlorhydrique dilué, évapore l'éthanol, extrait par de l'acétate d'éthyle, lave à l'eau, sèche sur sulfate de magnésium et évapore. Le solide obtenu est chromatographié sur el de silice en éluant par de l'acétate d'éthyle. Après recristallisation par dissolution dans le dichlorométhane et addition du minimum d'éther isopropylique, on obtient 477 mg du produit attendu.
Rendement 35 % ;
Fc = 142-144° C ;
alpha D = +27,2 (c = 1,14 ; méthanol).
$C_{16}H_{20}N_2O_3$ :
Analyse : Calculé : C = 66,65 H = 6,99 N = 9,72
Trouvé : C = 66,49 H = 6,92 N = 9,59.

Le même composé peut être préparé par un mode opératoire différent, décrit ci-après :

On chaufre sous agitation magnétique pendant une nuit à 120° C dans 15 ml de DMF 1,12 g du diol obtenu à l'étape 3 en présence de 1,1 g de carbonate de potassium solide. On évapore le solvant à 50° C sous vide de la pompe à palettes, ajoute de l'eau, extrait par 3 volumes d'acétate d'éthyle, lave une fois par de l'eau, de l'eau saline et sèche sur sulfate de magnésium. Après évaporation du solvant, on isole 820 mg d'un produit solide. On le purifie sur une colonne de gel de silice prééquilibrée dans l'acétate d'éthyle.

En éluant par le même solvant, on obtient le composé attendu sous forme d'un solide (600 mg) homogène en CCM.
Rendement : 68 % ;
alpha D du composé non recristallisé = +26,5
(c = 0,83 ; méthanol).

5) - (S) (+) [Diméthyl-1,1 (indolyl-3)-2 éthyl]-3 tosyloxyméthyl-5 oxazolidin-1,3 one-2.

On dissout le composé préparé à l'étape précédente (1,155 g) dans 6 ml de pyridine puis on ajoute à 0° C un équivalent de chlorure de tosyle (762 mg) et on conserve au réfrigérateur pendant 3 jours. On verse dans l'eau glacée, extrait par 4 volumes de chlorure de méthylène, lave 2 fois par 100 ml d'une solution glacée d'acide chlorhydrique 2N puis par de l'eau. Après séchage sur sulfate de sodium, on évapore ; le résidu est utilisé tel quel pour la réaction ultérieure.

6) - (S) (+) [Diméthyl-1,1 (indolyl-3)-2 éthyl]-3 [(N-tosyl indolyl-4) oxyméthyl]-5 oxazolidin-1,3 one-2.

On dissout sous azote dans 4 ml de DMF 643 mg de N-tosyl hydroxy-4 indole préparé par des méthodes connues. On ajoute 125 mg d'hydrure de sodium à 55 % et laisse sous agitation à température ambiante pendant 15 minutes, on ajoute 1 g du tosylate préparé à l'étape précédente dans 6 ml de DMF puis, après 1 heure sous agitation à température ambiante, on chauffe à 50° C pendant 90 minutes. On verse le mélange réactionnel sur de la glace, extrait par 4 volumes d'acétate d'éthyle, lave 3 fois par une solution de carbonate de sodium puis par de l'eau et de l'eau saline. On sèche sur sulfate de magnésium et évapore. Le résidu est chromatographié sur gel de silice (2 fois) en éluant par le mélange pentane-acétate d'éthyle (40/60 ; v/v). Le produit est recristallisé du mélange chlorure de méthylène-méthanol.
On obtient 270 mg ;
Rendement 22 % ;
alpha D = +46,5 (c = 0,6 ; chloroforme) à 24° C ;
Fc = 141-143° C
$C_{31}H_{31}N_3O_5S$ :
Analyse : Calculé : C = 66,77 H = 5,60 N = 7,54
Trouvé : C = 66,38 H = 5,50 N = 7,37

7) - (S) (+) [Diméthyl-1,1 (indolyl-3)-2 éthyl]-3 [(indolyl-4) oxyméthyl]-5 oxazolidin-1,3 one-2.

On chauffe au reflux sous azote dans un mélange eau-dioxanne-éthanol (3/3/7 ; v/v/v) 240 mg du composé obtenu précédemment en présence de 75 mg de soude pendant 48 heures. On évapore les solvants organiques, extrait par de l'acétate d'éthyle, lave par une solution de carbonate de sodium, de l'eau, de l'eau saline et sèche sur sulfate de magnésium. L'huile obtenue (187 mg) est chromatographiée sur gel de silice ; en éluant par de l'acétate d'éthyle, on obtient 127 mg de l'oxazolidinone attendue. Après trituration dans le mélange de dichlorométhane-éther isopropylique, on recueille 107 mg.
Rendement 63 % ;
Fc = 202-204° C ;

alpha D = +56 (c = 0,196 ; chloroforme-diméthylformamide ; 1/1 ; v/v)
$C_{24}H_{25}N_3O_3$ :
Analyse : Calculé : C = 71,44 H = 6,25 N = 10,41
Trouvé : C = 70,42 H = 6,20 N = 10,14.

On peut préparer ce composé à partir du produit obtenu à l'étape (5), sans isoler le dérivé N-tosylé de l'étape (6).

On dissout sous azote dans 4 ml de DMF anhydre à 20° C 643 mg de N-tosyl hydroxy-4 indole. On ajoute 120 mg d'hydrure de sodium à 55 % et laisse sous agitation à température ambiante pendant 30 minutes. On ajoute alors 1 g de tosylate, préparé à l'étape 5, agite pendant 3 heures à température ambiante puis une nuit à 50° C. On verse le mélange réactionnel sur de la glace, ajoute de l'eau et extrait par 4 volumes d'acétate d'éthyle, lave 3 fois par une solution de carbonate de sodium, puis par de l'eau et de l'eau saline. Le résidu est chromatographié sur gel de silice et on élue par le mélange hexane-acétate d'éthyle (50/50 ; v/v). On isole une mousse (900 mg) qui se solidifie par adjonction de chlorure de méthylène. Cette mousse contient 3 produits :

- (S) (+) [diméthyl-1,1 (indolyl-3)-2 éthyl]-3 (N-tosyl indolyl-4) oxyméthyl-5 oxazolidin-2 one ;
- (S) (+) [diméthyl-1,1 (N-tosyl indolyl-3)-2 éthyl]-3 [N-tosyl indolyl-4) oxyméthyl]-5 oxazolidin-1,3 one-2 ;
- et le produit attendu.

On dissout les 900 mg obtenus dans le méthoxy-2 éthanol (25 ml), ajoute 280 mg de NaOH dans 10 ml d'eau et chauffe pendant 4 heures à 80° C sous agitation magnétique. Après refroidissement, on ajoute de l'eau, extrait par 4 volumes d'acétate d'éthyle, lave une fois par une solution de carbonate de sodium, puis par de l'eau et de l'eau saline. Après séchage ($MgSO_4$) et évaporation du solvant organique sous vide, on isole un solide blanc que l'on purifie par filtration sur une colonne de gel de silice en éluant par un mélange de pentane et d'acétate d'éthyle (40/60 ; v/v). Le produit ainsi isolé, non recristallisé, présente les caractéristiques suivantes :
Poids 640 mg ;
Rendement : 67 % ;
Fc : 193-202° C ;
alpha D = +54,3 (c = 0,5 ; DMF/chloroforme ; 50/50 ; v/v).

8) - (S) (-) CM 40441.

On chauffe à reflux 101 mg de l'oxazolidinone obtenue à l'étape précédente dans 5 ml de méthoxyéthanol, on ajoute 2 ml de soude à 30 % et maintient le reflux pendant 3 jours. On évapore le méthoxyéthanol, ajoute de l'eau, acidifie par une solution de sulfate acide de potassium puis alcalinise par du carbonate de sodium. Après extraction par de l'acétate d'éthyle puis séchage sur sulfate de sodium et évaporation, on isole une mousse (158 mg). On chromatographie sur gel de silice en éluant par le mélange acétate d'éthyleméthanol (80/20 ; v/v). Le produit est isolé sous forme de mousse.
Poids : 67 mg ;
Rendement : 72 % ;
alpha D = -17,2 plus ou moins 1,2 (c = 0,46 ; chloroforme).

9) - Hémifumarate de (S) (-) CM 40441.

On prépare ce sel par action d'un équivalent d'acide fumarique sur 905 mg du composé obtenu à l'étape précédente, dans l'éthanol absolu. On obtient 586 mg du sel attendu.
Fc = 152-156° C ;
alpha D = -17,6 (c = 0,89 ; diméthylformamide) ;
Pouvoir rotatoire mesuré en lumière polarisée à 400 nm : -44,6 (c = 0,89 ; diméthylformamide).

<u>Exemple 3</u>

(S) (-) [ Diméthyl-1,1 (indolyl-3)-2 éthylamino]-3 [(indolyl-4) oxy]-1 propanol-2 ; (S) (-) CM 40 441.
Ce composé peut être préparé selon une variante de la méthode décrite à l'exemple 2.

1) - (S) (+) [(Diméthyl-1,1 (indolyl-3)-2 éthylamino]-3 0-isopropylidène-1,2 propanediol-1,2.

Ce composé est préparé comme à l'exemple 2, étape 1.

2) - (S) (+) [(Diméthyl-1,1 (indolyl-3)-2 éthyl]-3 hydroxyméthyl-5 oxazolidin-1,3 one-2.

A une solution de 6 g du composé obtenu à l'étape précédente dans 100 ml de dioxanne, maintenue à 12° C, on ajoute 3,9 g de chloroformiate de méthyle en 5 minutes et on laisse sous agitation à 12° C pendant 90 minutes. On ajoute alors une solution de 5,6 g de carbonate de potassium dans 15 ml d'eau et agite pendant 60 minutes à 10° C. On ajoute à nouveau 2 g de chloroformiate de méthyle puis laisse remonter la température vers 15-18° C. On additionne 3,5 g de carbonate de potassium solide et agite pendant 60 minutes à 20° C. Après avoir ajouté 1 g de chloroformiate de méthyle supplémentaire, on laisse 1 heure sous agitation à 20° C et ajoute une solution de carbonate de potassium en excès. On extrait par 3 volumes d'acétate d'éthyle, lave par de l'eau, de l'eau saline, sèche sur sulfate de magnésium et évapore.

Le résidu obtenu (huile, environ 7,4 g) correspond à [diméthyl-1,1 (indolyl-3)-2 (N-méthoxycarbonyl)-éthylamino]-3 0-isopropylidène-1,2 propanediol-1,2.

alpha D = +8,6 (c = 0,52 ; méthanol).

Ce résidu est placé dans 35 ml de méthanol et traité par 8 ml d'une solution d'acide chlorhydrique 0,1N à température ambiante pendant 3 heures puis à 40° C pendant 2 heures et demie, jusqu'à la disparition totale du produit de départ. On ajoute de l'eau carbonatée, évapore le méthanol à l'évaporateur rotatif à 40° C sous vide, puis on extrait par 3 volumes d'acétate d'éthyle, lave par de l'eau, de l'eau saline, sèche sur sulfate de magnasium et évapore le solvant pour obtenir une huile qui correspond à diméthyl-1,1 (indolyl-3)-2 (N-méthoxycarbonyl) éthylamino-3 propanediol-1,2.

alpha D = -36,0 (c = 0,39 ; méthanol).

Ce produit (6,4 g) est chauffé dans 100 ml de DMF à 120° C en présence de 6,4 g de carbonate de potassium sous agitation pendant une nuit. Le solvant est évaporé à 40° C, sous vide, à l'évaporateur rotatif. On ajoute de l'eau, extrait par 3 volumes d'acétate d'éthyle, lave par de l'eau, de l'eau saline, sèche sur sulfate de magnésium et évapore le solvant sous vide. Le solide attendu cristallise, on le filtre pour obtenir 2,4 g.

alpha D = +27,3 (c = 0,68 ; méthanol).

Les eaux mères sont chromatographiées sur gel de silice ; en éluant par de l'acétate d'éthyle pur, on obtient une seconde fraction du produit attendu, homogène en CCM : 2,61 g.

alpha D = +26,4 (c = 0,76 ; méthanol) ;

Rendement total des 3 réactions : 87 %.

3) - (S) (+) [Diméthyl-1,1 (indolyl-3)-2 éthyl]-3 tosyloxyméthyl-5 oxazolidin-1,3 one-2.

On dissout 4,93 g de l'alcool obtenu à l'étape précédente dans 29 ml de pyridine anhydre puis on ajoute 3,44 g de chlorure de tosyle solide à la solution refroidie à -10° C, après quelques instants d'agitation, on conserve 3 jours au refrigérateur. On verse alors le mélange dans une solution de 100 ml d'acide chlorhydrique normal contenant de la glace, puis on extrait par 3 volumes d'acétate d'éthyle, lave la solution organique 2 fois par une solution glacée d'acide chlorhydrique normal puis par de l'eau et de l'eau saline. Après séchage sur sulfate de magnésium et évaporation à l'évaporateur rotatif à 30° C, on isole le tosylate attendu qui est purifié par filtration sur une colonne de gel de silice prééquilibrée dans du chlorure de méthylène, on élue par du chlorure de méthylène. Après évaporation, on isole une mousse : 6,58 g.

Rendement : 90,2 % ;

alpha D = +42,5 (+1 ou -1) (c = 0,68 à 0,84 ; chloroforme).

4) - (S) (+) [Diméthyl-1,1 (indolyl-3)-2 éthyl]-3 [(N-tosyl indolyl-4) oxyméthyl]-5 oxazolidin-1,3 one-2.

On dissout dans 4 ml de DMF anhydre, sous atmosphère d'azote, 643 mg de N-tosyl hydroxy-4 indole, à 20° C, on ajoute 120 mg d'hydrure de sodium à 55 % en dispersion dans de l'huile et agite lentement pendant 30 minutes. On ajoute ensuite 1 g du produit obtenu à l'étape précédente, agite à température ambiante pendant 3 heures puis une nuit à 50° C. On verse alors dans un mélange eau-glace, extrait par 3 volumes d'acétate d'éthyle, lave par de l'eau, de l'eau saline, sèche sur sulfate de magnésium et évapore le solvant à l'évaporateur rotatif. Le résidu est chromatographié sur gel de silice éluant par le mélange hexane-acétate d'éthyle (1/1 ; v/v) puis l'acétate d'éthyle pur. La première fraction est homogène en CCM (éluant : cyclohexane-acétate d'éthyle : 1/1 ; v/v), la seconde fraction est inhomogène en CCM (éluant : chloroforme). On recueille ainsi 900 mg du produit attendu.

Rendement : 72 %.

5) - (S) (+) [Diméthyl-1,1 (indolyl-3)-2 éthyl]-3 [(indolyl-4) oxyméthyl]-5 oxazolidin-1,3 one-2.

On dissout les 900 mg du produit obtenu précédemment dans 25 ml de méthoxy-2 éthanol, ajoute une solution de 280 mg de soude dans 10 ml d'eau et agite à 80 °C sous atmosphère d'azote jusqu'à disparition du produit de départ, pendant environ 4 heures. On refroidit, ajoute de l'eau, extrait par 3 volumes d'acétate d'éthyle, lave par une solution de carbonate de sodium, par de l'eau, de l'eau saline et sèche sur sulfate de magnésium. Après évaporation du solvant à l'évaporateur rotatif, on isole un solide blanc : 640 mg.

Rendement : 94 %.

Ce produit est homogène en CCM. Il n'est pas recristallisé.

alpha D = +54,3 (c = 0,23 ; chloroforme-DMF ; 1/1 ; v/v) ;

Fc = 193-202 °C.

6) - (S) (-) CM 40441.

Ce produit est obtenu en procédant comme à l'exemple 2, étape 8.

## Revendications

**Revendications pour les Etats contractants suivants : BE, CH, DE, FR, GB, GR, IT, LI, LU, NL, SE**

**1.** Procéde de synthèse stéréospécifique de dérivés de l'indole de formule :

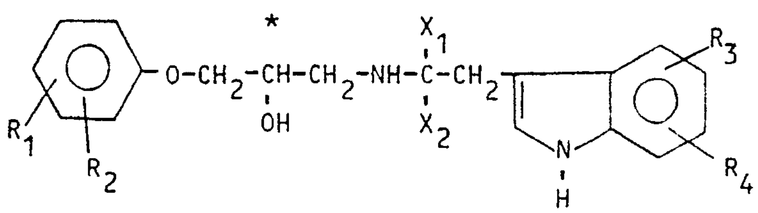

(I)

dans laquelle :

- l'atome C* a la configuration (R) ou (S) ;
- $X_1$ et $X_2$ désignent chacun indépendamment un atome d'hydrogène ou un alkyle droit ou ramifié comportant de 1 à 4 atomes de carbone;
- $R_1$ et $R_2$ désignent chacun indépendamment un atome d'hydrogène, un atome d'halogène, un alkyle droit ou ramifié comportant de 1 à 4 atomes de carbone, un alcoxy droit ou ramifié comportant de 1 à 4 atomes de carbone, un formyle, ou $R_1$ et $R_2$ pris ensemble avec le noyau benzénique auquel ils sont liés forment un noyau bicyclique choisi parmi ceux de formule :

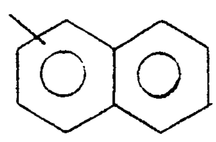 ; 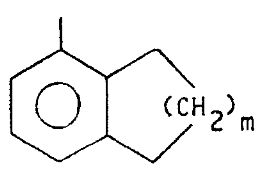 ; 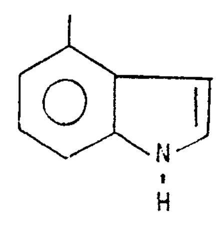

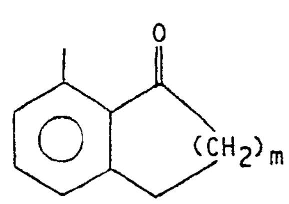 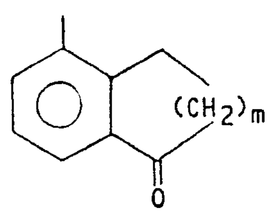

- $R_3$ et $R_4$ désignent chacun indépendamment un atome d'hydrogène, un atome d'halogène, un alkyle droit ou ramifié comportant de 1 à 4 atomes de carbone ou un alcoxy droit ou ramifié comportant de 1 à 4 atomes de carbone ;
- m représente un nombre entier compris entre 1 et 3 ;

et de leurs sels pharmaceutiquement acceptables avec les acides organiques ou minéraux ; caractérisé en ce que :

a) on condense le tosyloxy-3 O-isopropylidène-1,2 propanediol-1,2 de formule (II) sous forme optiquement pure :

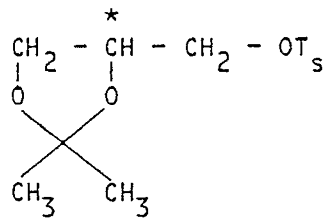

(II)

avec une amine primaire de formule (III)

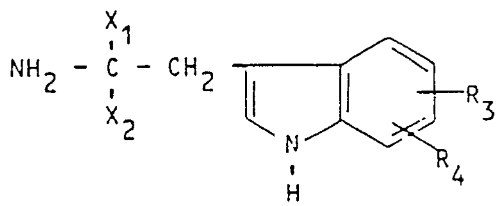

(III)

par chauffage dans un solvant inerte pour obtenir le dérivé de formule (IV) :

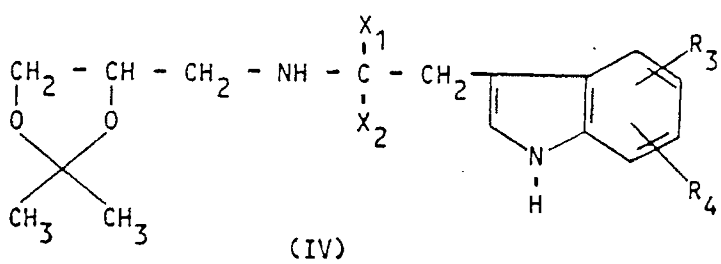

(IV)

b) dans un ordre quelconque, on effectue une hydrolyse en milieu acide du cycle dioxolanne du composé de formule (IV) et on substitue la fonction amine dudit composé par un groupement oxycarbonyle ROCO dans lequel R représente un groupe alkyle droit ou ramifié comportant de 1 à 4 atomes de carbone ou un benzyl, pour former le composé de formule (VI) :

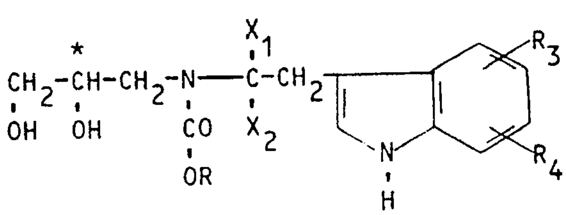

(VI)

c) on chauffe le composé (VI) en milieu basique pour préparer l'oxazolidinone de formule :

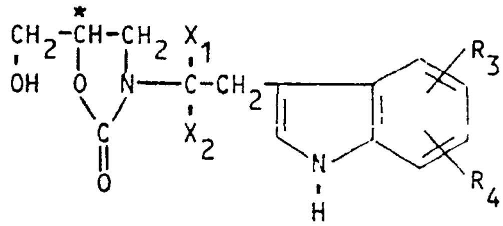

(VII)

d) on estérifie la fonction alcool du composé de formule (VII) par du chlorure de tosyle ou du chlorure de mésyle dans la pyridine ;
e) on fait réagir sur le composé de formule (VII) ainsi estérifié en présence d'un agent alcalin et dans un solvant approprié un phénol de formule (VIII)

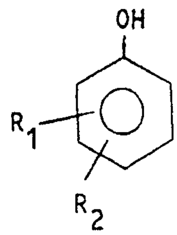

(VIII)

pour préparer le composé de formule (IX) :

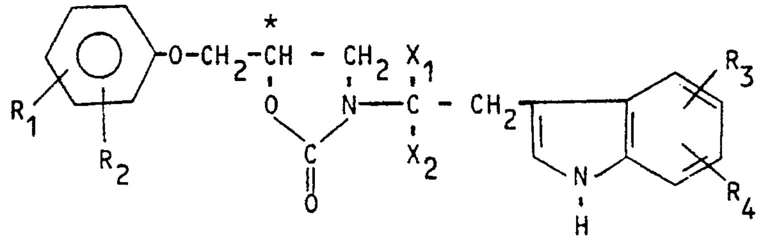

(IX)

f) on chauffe au reflux ledit composé de formule (IX) en milieu basique dans un solvant convenable en présence d'eau et on isole le composé de formule (I) obtenu ;
g) éventuellement, on transforme celui-ci en un de ses sels pharmaceutiquement acceptables, par action d'un acide minéral ou organique.

**2.** Procédé selon la revendication 1, caractérisé en ce que l'étape a) est réalisée avec une amine de formule (III) optiquement pure pour laquelle $X_1$ est différent de $X_2$.

**3.** Procédé selon la revendication 1, caractérisé en ce que l'étape a) est réalisée avec une amine de formule (III) dans laquelle $X_1$ est différent de $X_2$, sous forme racémique et en ce que la résolution du mélange d'épimères peut être effectuée après toute étape du procédé ou à la dernière étape.

**4.** Procédé selon l'une des revendications 1 ou 3, caractérisé en ce qu'à l'étape a) on utilise comme amine de formule (III) la d,l alpha méthyl tryptamine et à l'étape e) on utilise comme phénol de formule (VIII) l'orthocrésol.

**5.** Procédé selon la revendication 1, caractérisé en ce qu'à l'étape (a) on utilise comme amine de formule

(III) l'alpha, alpha diméthyl tryptamine, et à l'étape e) on utilise comme dérivé du phénol de formule (VIII) l'hydroxy-4 indole.

6. Procédé selon l'une quelconque des revendications précédentes, caractérisé en ce que le groupement oxycarbonyle ROCO, intervenant à l'étape b), est un groupement tert-butyloxycarbonyle ou méthyloxycarbonyle.

7. Procédé selon l'une quelconque des revendications 1 à 6, caractérisé en ce que le chauffage, à l'étape c), est effectué dans un mélange eau-alcool et doit être contrôlé.

8. Procédé selon l'une quelconque des revendications 1 à 6, caractérisé en ce que le chauffage, à l'étape c), est effectué dans un solvant non aqueux tel que le diméthylformamide.

9. Procédé selon l'une quelconque des revendications précédentes, caractérisé en ce que l'agent alcalin, intervenant à l'étape e), est choisi parmi la soude, la potasse, un alcoolate de sodium ou de potassium, l'hydrure de sodium.

10. Procédé selon l'une quelconque des revendications précédentes, caractérisé en ce que l'étape f) est réalisée dans un solvant convenable tel qu'un alcool, un glycol, un éther de glycol ou un solvant dipolaire tel que le disulfolane.

11. Dérivés de l'hydroxyméthyl-5 [(indolyl-3)-2 éthyl]-3 oxazolidin-1,3 one-2 de formule

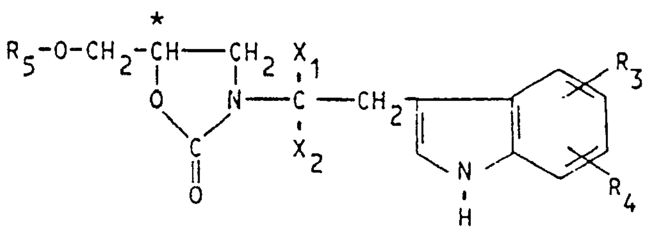

(X)

dans laquelle :

- l'atome $C^*$ a la configuration (R) ou (S) ;
- $R_5$ représente un atome d'hydrogène ou un groupe :

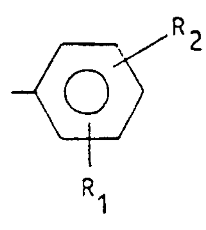

- $X_1$ et $X_2$ désignent chacun indépendamment un atome d'hydrogène ou un alkyle droit ou ramifié comportant de 1 à 4 atomes de carbone;
- $R_1$ et $R_2$ désignent chacun indépendamment un atome d'hydrogène, un atome d'halogène, un alkyle droit ou ramifié comportant de 1 à 4 atomes de carbone, un alcoxy droit ou ramifié comportant de 1 à 4 atomes de carbone, un formyle, ou $R_1$ et $R_2$ pris ensemble avec le noyau benzénique auquel ils sont liés forment un noyau bicyclique choisi parmi ceux de formule :

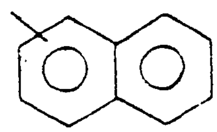 ; 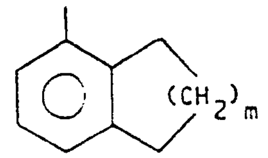 ; 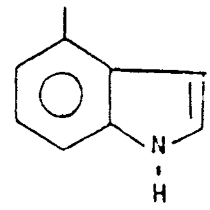

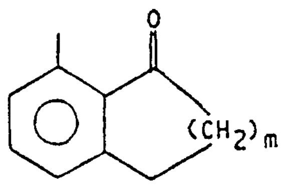 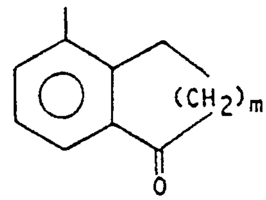

- $R_3$ et $R_4$ désignent chacun indépendamment un atome d'hydrogène, un atome d'halogène, un alkyle droit ou ramifié comportant de 1 à 4 atomes de carbone ou un alcoxy droit ou ramifié comportant de 1 à 4 atomes de carbone ;
- m représente un nombre entier compris entre 1 et 3.

**12.** Dérivés selon la revendication 11, de formule (X), dans laquelle $X_1$ est différent de $X_2$, le carbone portant $X_1$ et $X_2$ ayant la configuration (R) ou (S).

**13.** Dérivés selon la revendication 11, de formule (X) dans laquelle $X_1$ est différent de $X_2$ ; lesdits dérivés étant des mélanges d'épimères.

**14.** Dérivés selon la revendication 11, choisis parmi :
- le (S) (+) [ diméthyl-1,1 (indolyl-3)-2 ethyl] [(indolyl-4) oxyméthyl]-5 oxazolidin-1,3 one-2 ;
- le (2S,1'R) [(indolyl-3)-2 méthyl-1 éthyl]-3 (méthyl-2 phénoxy-méthyl)-5 oxazolidin-1,3 one-2 ;
- le (2S,1'S) [(indolyl-3)-2 méthyl-1 éthyl]-3 (méthyl-2 phénoxy-méthyl)-5 oxazolidin-1,3 one-2.

**Revendications pour les Etats contractants suivants : AT, ES**

**1.** Procéde de synthèse stéréospécifique de dérivés de l'indole de formule :

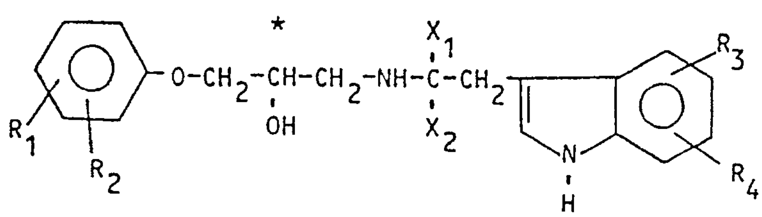

(I)

dans laquelle :
- l'atome C* a la configuration (R) ou (S) ;
- $X_1$ et $X_2$ désignent chacun indépendamment un atome d'hydrogène ou un alkyle droit ou ramifié comportant de 1 à 4 atomes de carbone;
- $R_1$ et $R_2$ désignent chacun indépendamment un atome d'hydrogène, un atome d'halogène, un alkyle droit ou ramifié comportant de 1 à 4 atomes de carbone, un alcoxy droit ou ramifié comportant de 1 à 4 atomes de carbone, un formyle, ou $R_1$ et $R_2$ pris ensemble avec le noyau benzénique auquel ils sont liés forment un noyau bicyclique choisi parmi ceux de formule :

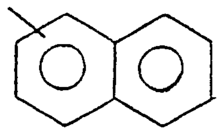 ; 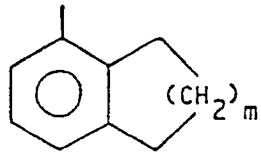 ; 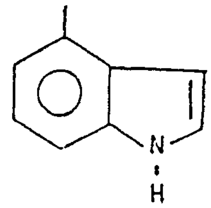

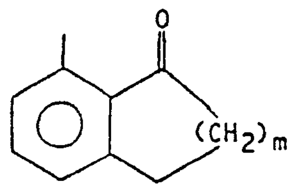 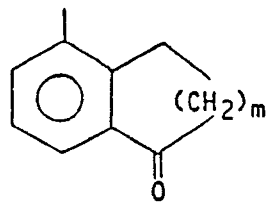

- $R_3$ et $R_4$ désignent chacun indépendamment un atome d'hydrogène, un atome d'halogène, un alkyle droit ou ramifié comportant de 1 à 4 atomes de carbone ou un alcoxy droit ou ramifié comportant de 1 à 4 atomes de carbone ;
- m représente un nombre entier compris entre 1 et 3 ; et de de leurs sels pharmaceutiquement acceptables avec les acides organiques ou minéraux ;

caractérisé en ce que :

a) on condense le tosyloxy-3 0-isopropylidène-l,2 propanediol-1,2 de formule (II) sous forme optiquement pure :

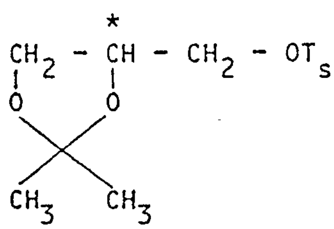

(II)

avec une amine primaire de formule (III)

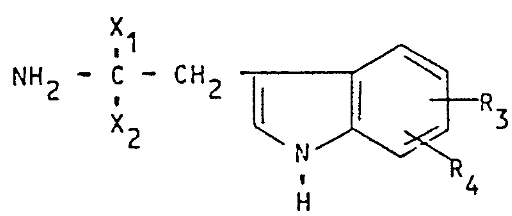

(III)

par chauffage dans un solvant inerte pour obtenir le dérivé de formule (IV) :

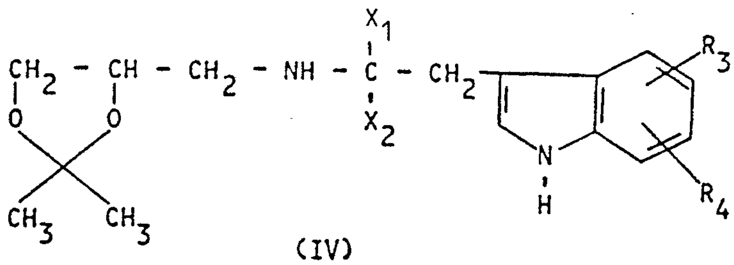

(IV)

b) dans un ordre quelconque, on effectue une hydrolyse en milieu acide du cycle dioxolanne du composé de formule (IV) et on substitue la fonction amine dudit composé par un groupement oxycarbonyle ROCO dans lequel R représente un groupe alkyle droit ou ramifié comportant de 1 à 4 atomes de carbone ou un benzyle, pour former le composé de formule (VI) :

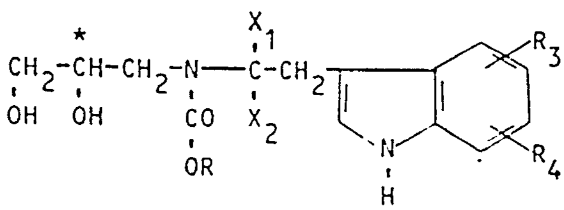

(VI)

c) on chauffe le composé (VI) en milieu basique pour préparer l'oxazolidinone de formule :

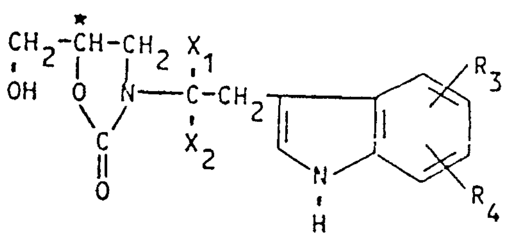

(VII)

d) on estérifie la fonction alcool du composé de formule (VII) par du chlorure de tosyle ou du chlorure de mésyle dans la pyridine ; e) on fait réagir sur le composé de formule (VII) ainsi estérifié en présence d'un agent alcalin et dans un solvant approprié un phénol de formule (VIII)

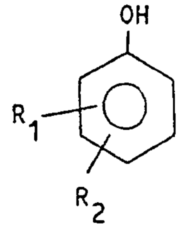

(VIII)

pour préparer le composé de formule (IX) :

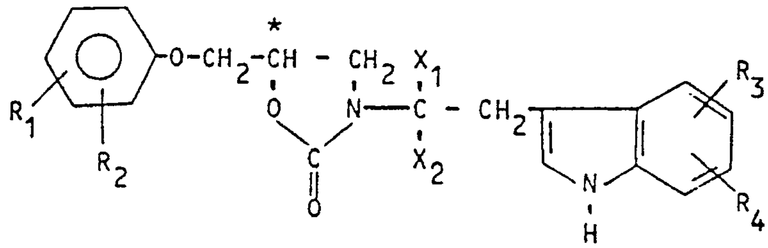

(IX)

f) on chauffe au reflux ledit composé de formule (IX) en milieu basique dans un solvant convenable en présence d'eau et on isole le composé de formule (I) obtenu ;

g) éventuellement, on transforme celui-ci en un de ses sels pharmaceutiquement acceptables, par action d'un acide minéral ou organique.

**2.** Procédé selon la revendication 1, caractérisé en ce que l'étape a) est réalisée avec une amine de formule (III) optiquement pure pour laquelle $X_1$ est différent de $X_2$.

**3.** Procédé selon la revendication 1, caractérisé en ce que l'étape a) est réalisée avec une amine de formule (III) dans laquelle $X_1$ est différent de $X_2$, sous forme racémique et en ce que la résolution du mélange d'épimères peut être effectuée après toute étape du procédé ou à la dernière étape.

**4.** Procédé selon l'une des revendications 1 ou 3, caractérisé en ce qu'à l'étape a) on utilise comme amine de formule (III) la d,l alpha méthyl tryptamine et à l'étape e) on utilise comme phénol de formule (VIII) l'orthocrésol.

**5.** Procédé selon la revendication 1, caractérisé en ce qu'à l'étape (a) on utilise comme amine de formule (III) l'alpha, alpha diméthyl tryptamine, et à l'étape e) on utilise comme dérivé du phénol de formule (VIII) l'hydroxy-4 indole.

**6.** Procédé selon l'une quelconque des revendications précédentes, caractérisé en ce que le groupement oxycarbonyle ROCO, intervenant à l'étape b), est un groupement tert-butyloxycarbonyle ou méthyloxycarbonyle.

**7.** Procédé selon l'une quelconque des revendications 1 à 6, caractérisé en ce que le chauffage, à l'étape c), est effectué dans un mélange eau-alcool et doit être contrôlé.

**8.** Procédé selon l'une quelconque des revendications 1 à 6, caractérisé en ce que le chauffage, à l'étape c), est effectué dans un solvant non aqueux tel que le diméthylformamide.

**9.** Procédé selon l'une quelconque des revendications précédentes, caractérisé en ce que l'agent alcalin, intervenant à l'étape e), est choisi parmi la soude, la potasse, un alcoolate de sodium ou de potassium, l'hydrure de sodium.

**10.** Procédé selon l'une quelconque des revendications précédentes, caractérisé en ce que l'étape f) est réalisée dans un solvant convenable tel qu'un alcool, un glycol, un éther de glycol ou un solvant dipolaire tel que le disulfolane.

**11.** Procédé pour la préparation de dérivés de l'hydroxyméthyl-5 [(indolyl-3)-2 éthyl]-3 oxazolidin-1,3 one-2 de formule

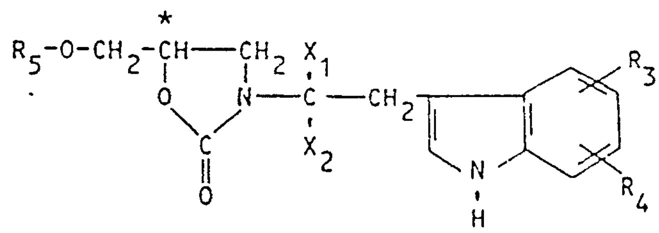

(X)

dans laquelle :

- l'atome C* a la configuration (R) ou (S) ;
- $R_5$ représente un atome d'hydrogène ou un groupe :

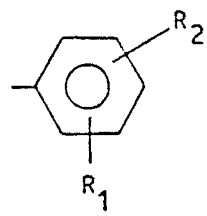

- $X_1$ et $X_2$ désignent chacun indépendamment un atome d'hydrogène ou un alkyle droit ou ramifié comportant de 1 à 4 atomes de carbone;
- $R_1$ et $R_2$ désignent chacun indépendamment un atome d'hydrogène, un atome d'halogène, un alkyle droit ou ramifié comportant de 1 à 4 atomes de carbone, un alcoxy droit ou ramifié comportant de 1 à 4 atomes de carbone, un formyle, ou $R_1$ et $R_2$ pris ensemble avec le noyau benzénique auquel ils sont liés forment un noyau bicyclique choisi parmi ceux de formule :

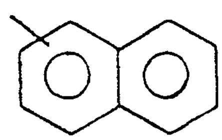
;
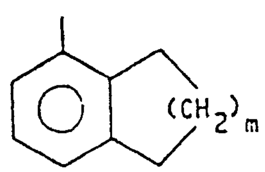
;
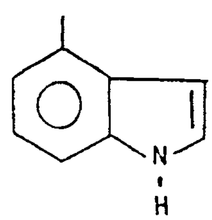

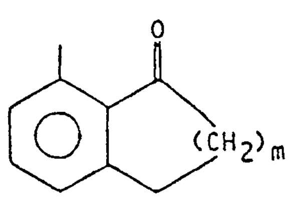

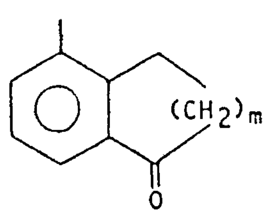

- $R_3$ et $R_4$ désignent chacun indépendamment un atome d'hydrogène, un atome d'halogène, un alkyle droit ou ramifié comportant de 1 à 4 atomes de carbone ou un alcoxy droit ou ramifié comportant de 1 à 4 atomes de carbone ;
- m représente un nombre entier compris entre 1 et 3,

caractérisé en ce que :

a) on condense le tosyloxy-3 0-isopropylidène-1,2 propanediol-1,2 de formule (II) sous forme optiquement pure :

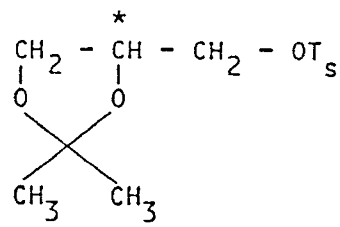

(II)

avec une amine primaire de formule (III)

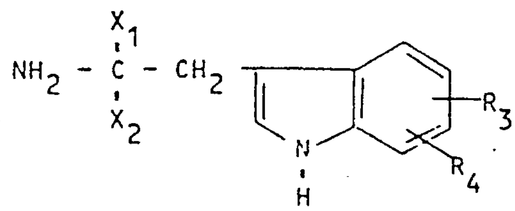

(III)

par chauffage dans un solvant inerte pour obtenir le dérivé de formule (IV) :

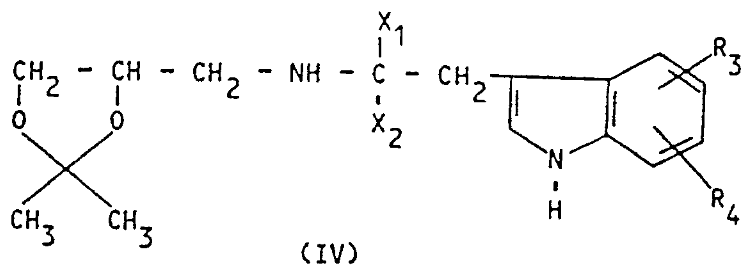

(IV)

b) dans un ordre quelconque, on effectue une hydrolyse en milieu acide du cycle dioxolanne du composé de formule (IV) et on substitue la fonction amine dudit composé par un groupement oxycarbonyle ROCO dans lequel R représente un groupe alkyle droit ou ramifié comportant de 1 à 4 atomes de carbone ou un benzyle, pour former le composé de formule (VI) :

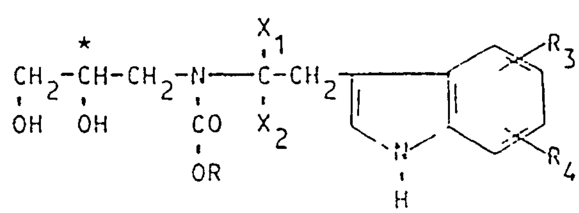

(VI)

c) on chauffe le composé (VI) en milieu basique pour préparer l'oxazolidinone de formule :

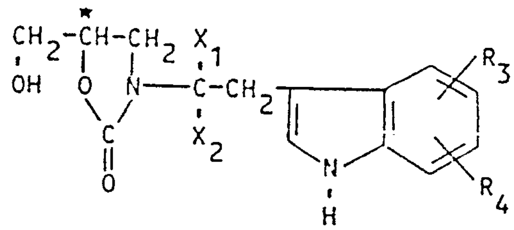

(X)

dans laquelle $R_5$ = H ;
et en ce que, pour préparer le composé de formule (X) dans laquelle $R_5$ représente un groupe

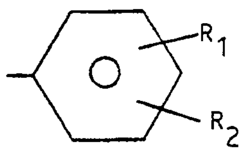

d) on estérifie la fonction alcool du composé de formule (X) dans laquelle $R_5$ = H par du chlorure de tosyle ou du chlorure de mésyle dans la pyridine ;
et
e) on fait réagir sur le composé ainsi estérifié, en présence d'un agent alcalin et dans un solvant approprié un phénol de formule (VIII)

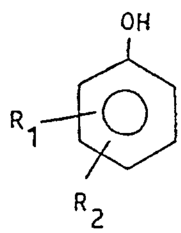

(VIII)

**12.** Procédé selon la revendication 11, caractérisé en ce que l'étape a) est réalisée avec une amine de formule (III) dans laquelle $X_1$ est différent de $X_2$, le carbone portant $X_1$ et $X_2$ ayant la configuration (R) ou (S).

**13.** Procédé selon la revendication 11, caractérisé en ce que l'étape a) est réalisée avec une amine de formule (III) dans laquelle $X_1$ est différent de $X_2$, sous forme racémique et en ce que les dérivés de formule (X) sont obtenus sous forme de mélanges d'épimères.

**14.** Procédé selon la revendication 11, caractérisé en ce qu'il est mis en oeuvre pour préparer :
- le (S) (+) [diméthyl-1,1 (indolyl-3)-2 éthyl]-3[(indolyl-4) oxyméthyl]-5 oxazolidin-1,3 one-2 ;
- le (2S,1'R) [(indolyl-3)-2 méthyl-1 éthyl]-3 (méthyl-2 phénoxyméthyl)-5 oxazolidin-1,3 one-2 ;
- le (2S,1'S) [(indolyl-3)-2 méthyl-1 éthyl]-3 (méthyl-2 phénoxyméthyl)-5 oxazolidin-1,3 one-2

en utilisant les réactifs de formule (III) et (VIII) adéquats.

## Claims

### Claims for the following Contracting States : BE, CH, DE, FR, GB, GR, IT, LI, LU, NL, SE

**1.** Process for the stereospecific synthesis of indole derivatives of the formula:

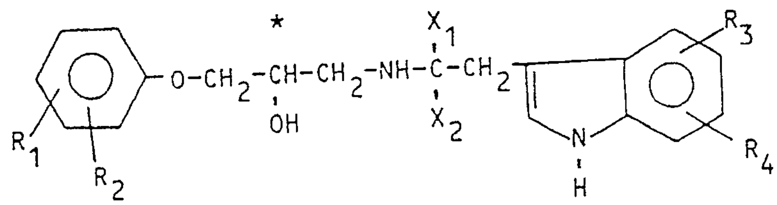

(I)

in which:

- the C* atom has the (R) or (S) configuration;
- $X_1$ and $X_2$ each independently denote a hydrogen atom or straight or branched alkyl with 1 to 4 carbon atoms;
- $R_1$ and $R_2$ each independently denote a hydrogen atom, a halogen atom, a straight or branched alkyl with 1 to 4 carbon atoms, a straight or branched alkoxy with 1 to 4 carbon atoms, a formyl, or $R_1$ and $R_2$, taken together with the benzene nucleus to which they are bonded, form an optionally substituted bicyclic nucleus selected from those of formula:

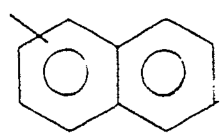 ; 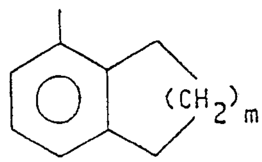 ; 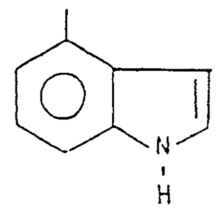

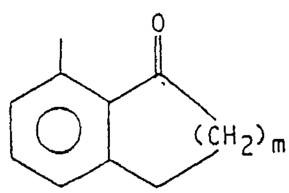 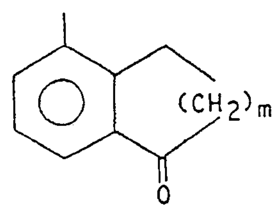

- $R_3$ and $R_4$ each independently denote a hydrogen atom, a halogen atom, a straight or branched alkyl with 1 to 4 carbon atoms or a straight or branched alkoxy with 1 to 4 carbon atoms;
- m represents an integer between 1 and 3.

characterized in that it comprises the steps of:

a) condensing 3-tosyloxy-1,2-0-isopropylidenepropane-1,2-diol of the formula (II) in an optically pure form:

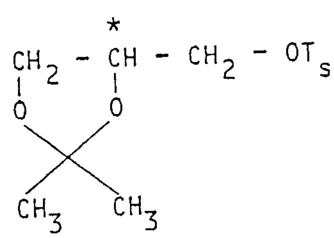

(II)

with a primary amine of the formula (III),

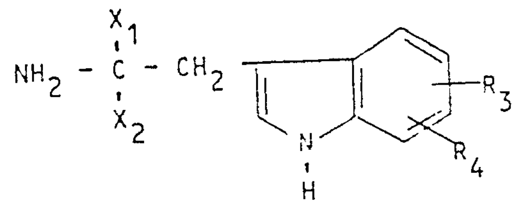

(III)

by heating in an inert solvent, to give the derivative of the formula (IV):

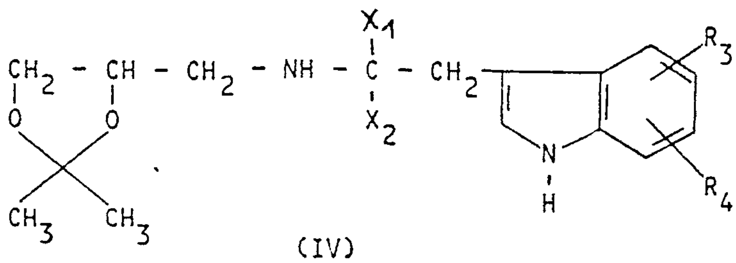

(IV)

b) carrying out in any order the hydrolysis of the dioxolan ring of the compound of formula (IV) in an acid medium and the substitution of the amine functional group of the said compound by an oxycarbonyl group ROCO, in which R represents a lower straight or branched alkyl with 1 to 4 carbon atoms or a benzyl, to form the compound of the formula (VI) :

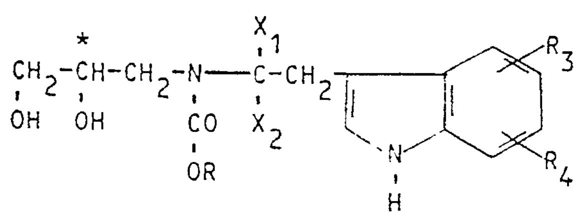

(VI)

c) heating the compound (VI) in a basic medium in order to prepare the oxazolidinone of the formula:

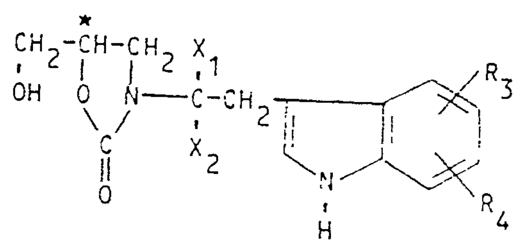

(VII)

d) esterifying the alcohol functional group of the compound of the formula (VII) with tosyl chloride or mesyl chloride in pyridine;

e) reacting the compound of the formula (VII) esterified in this way, in the presence of an alkaline agent and in an appropriate solvent, with a phenol of the formula (VIII):

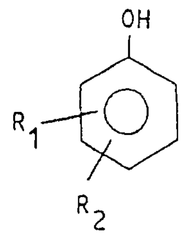

(VIII)

in order to prepare the compound of the formula (IX):

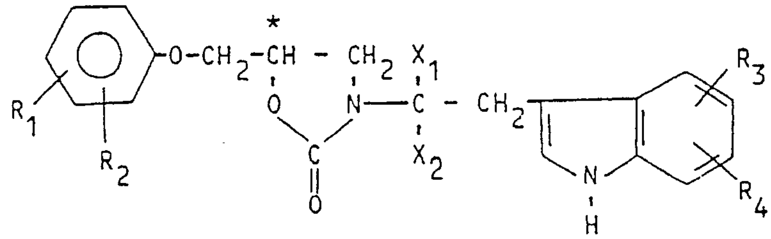

(IX)

f) heating the said compound of the formula (IX) under reflux in a basic medium, in a solvent, in the presence of water, and isolating the resulting compound of the formula (I);

g) if appropriate, converting the latter to one of its pharmaceutically acceptable salts by reaction with a mineral or organic acid.

2. Process according to claim 1, characterized in that step a) is carried out with an optically pure amine of the formula (III) in which $X_1$ is different from $X_2$.

3. Process according to claim 1, characterized in that step a) is carried out with an amine of the formula (III) in which $X_1$ is different from $X_2$, in the racemic form, and in that the mixture of epimers can be resolved after any step of the process or in the final step.

4. Process according to one of claims 1 or 3, characterized in that the amine of the formula (III) used in step a) is d,l-α-methyltryptamine and the phenol of the formula (VIII) used in step e) is orthocresol.

5. Process according to claim 1, characterized in that the amine of the formula (III) used in step a) is α,α-dimethyltryptamine and the phenol derivative of the formula (VIII) used in step e) is 4-hydroxyindole.

6. Process according to any one of the preceding claims, characterized in that the oxycarbonyl group ROCO involved in step b) is a tert.-butoxycarbonyl or methoxycarbonyl group.

7. Process according to any one of claims 1 to 6, characterized in that the heating in step c) is carried out in a water/alcohol mixture and must be controlled.

8. Process according to any one of claims 1 to 6, characterized in that the heating in step c) is carried out in a non-aqueous solvent, such as dimethylformamide.

9. Process according to any one of the preceding claims, characterized in that the alkaline agent involved in step e) is selected from the group consisting of sodium hydroxide, potassium hydroxide, a sodium or potassium alcoholate and sodium hydride.

10. Process according to any one of the preceding claims, characterized in that step f) is carried out in a suitable solvent such as an alcohol, a glycol, a glycol ether or a dipolar solvent such as disulfolan.

**11.** 5-Hydroxymethyl-3-[2-(indol-3-yl)ethyl]-1,3-oxazolidin-2-one derivatives of the formula:

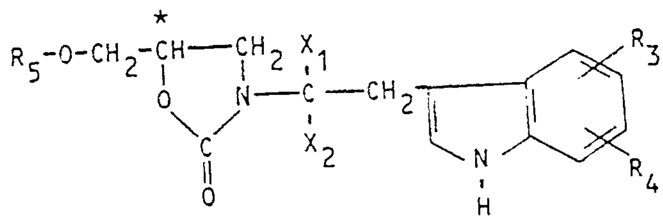

(X)

in which:

- the C* atom has the (R) or (S) configuration;
- $R_5$ represents a hydrogen atom or a group:

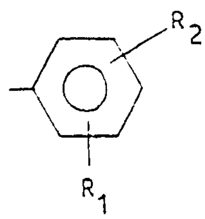

- $X_1$ and $X_2$ each independently denote a hydrogen atom or straight or branched alkyl with 1 to 4 carbon atoms;
- $R_1$ and $R_2$ each independently denote a hydrogen atom, a halogen atom, a straight or branched alkyl with 1 to 4 carbon atoms, a straight or branched alkoxy with 1 to 4 carbon atoms, a formyl, or $R_1$ and $R_2$, taken together with the benzene nucleus to which they are bonded, form a bicyclic nucleus selected from those of formula :

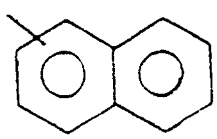 ; 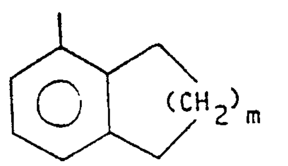 ; 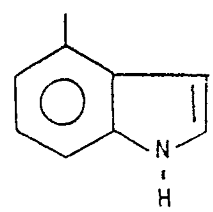

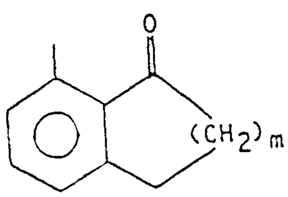 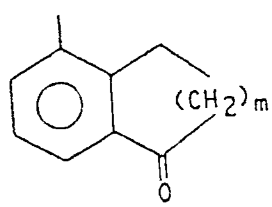

- $R_3$ and $R_4$ each independently denote a hydrogen atom, a halogen atom, a straight or branched alkyl with 1 to 4 carbon atoms or a straight or branched alkoxy with 1 to 4 carbon atoms;
- m represents an integer between 1 and 3.

**12.** Derivatives according to claim 22, of the formula (X), in which $X_1$ is different from $X_2$, the carbon carrying $X_1$ and $X_2$ having the configuration (R) or (S).

**13.** Derivatives according to claim 11, of the formula (X), in which $X_1$ is different from $X_2$, said derivatives being mixtures of epimers.

**14.** Derivatives according to claim 11, selected from:
- (S)(+)-3-[1,1-dimethyl-2-(indol-3-yl)ethyl]-5-[(indol-4-yl)oxymethyl]-1,3-oxazolidin-2-one;
- (2S,1'R)-3-[(2-indol-3-yl)-1-methylethyl]-5-(2-methylphenoxymethyl)-1,3-oxazolidin-2-one;
- (2S,1'S)-3-[2-(indol-3-yl)-1-methylethyl]-5-(2-methylphenoxymethyl)-1,3-oxazolidin-2-one.

**Claims for the following Contracting States : AT, ES**

**1.** Process for the stereospecific synthesis of indole derivatives of the formula:

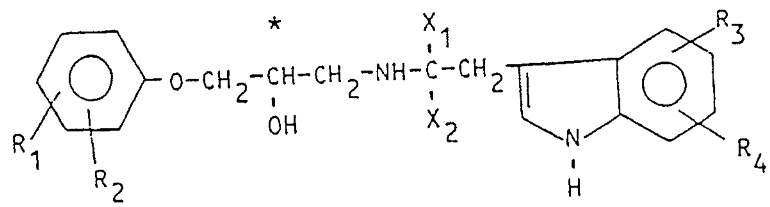

(I)

in which:
- the $C^*$ atom has the (R) or (S) configuration;
- $X_1$ and $X_2$ each independently denote a hydrogen atom or straight or branched alkyl with 1 to 4 carbon atoms;
- $R_1$ and $R_2$ each independently denote a hydrogen atom, a halogen atom, a straight or branched alkyl with 1 to 4 carbon atoms, a straight or branched alkoxy with 1 to 4 carbon atoms, a formyl, or $R_1$ and $R_2$, taken together with the benzene nucleus to which they are bonded, form an optionally substituted bicyclic nucleus selected from those of formula:

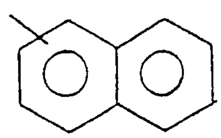 ; 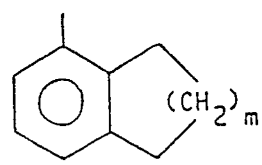 ; 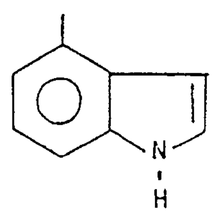

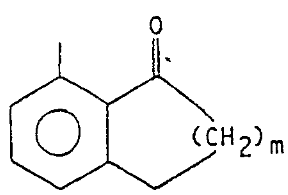 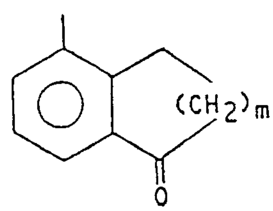

- $R_3$ and $R_4$ each independently denote a hydrogen atom, a halogen atom, a straight or branched alkyl with 1 to 4 carbon atoms or a straight or branched alkoxy with 1 to 4 carbon atoms;
- m represents an integer between 1 and 3, and their pharmaceutically acceptable salts with organic or mineral acids,

characterized in that it comprises the steps of

a) condensing 3-tosyloxy-1,2-0-isopropylidenepropane-1,2-diol of the formula (II) in an optically pure form:

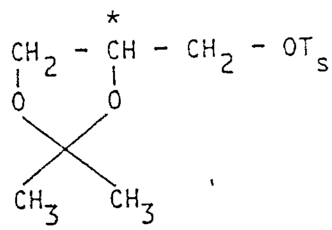

(II)

with a primary amine of the formula (III),

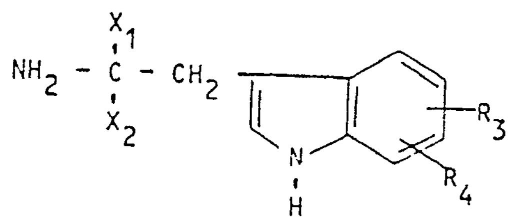

(III)

by heating in an inert solvent, to give the derivative of the formula (IV):

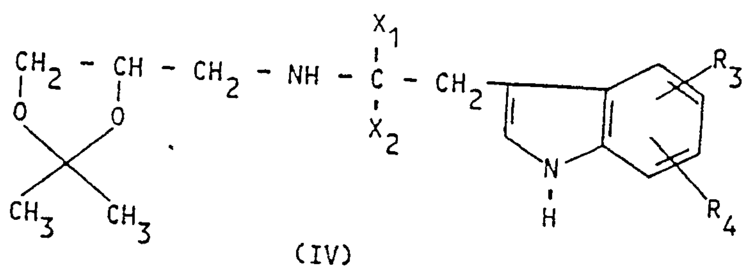

(IV)

b) carrying out in any order the hydrolysis of the dioxolan ring of the compound of formula (IV) in an acid medium and the substitution of the amine functional group of the said compound by an oxycarbonyl group ROCO, in which R represents a straight or branched alkyl with 1 to 4 carbon atoms or a benzyl, to form the compound of the formula (VI) :

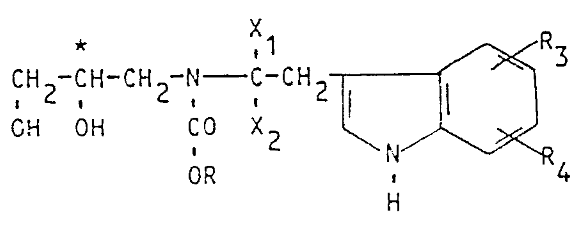

(VI)

c) heating the compound (VI) in a basic medium in order to prepare the oxazolidinone of the formula:

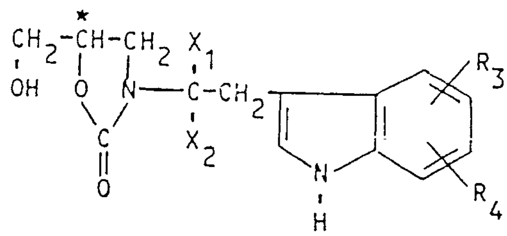

(VII)

d) esterifying the alcohol functional group of the compound of the formula (VII) with tosyl chloride or mesyl chloride in pyridine;

e) reacting the compound of the formula (VII) esterified in this way, in the presence of an alkaline agent and in an appropriate solvent, with a phenol of the formula (VIII):

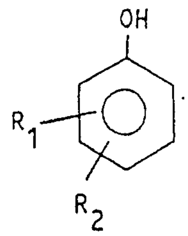

(VIII)

in order to prepare the compound of the formula (IX):

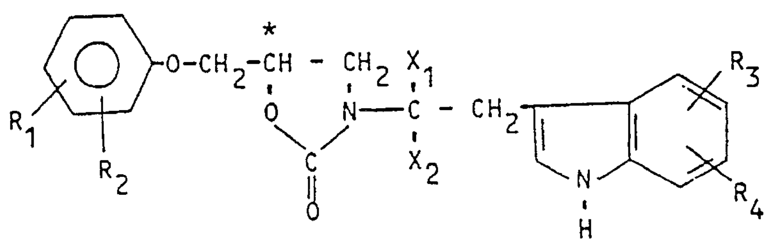

(IX)

f) heating the said compound of the formula (IX) under reflux in a basic medium, in a solvent, in the presence of water, and isolating the resulting compound of the formula (I);

g) if appropriate, converting the latter to one of its pharmaceutically acceptable salts by reaction with a mineral or organic acid.

**2.** Process according to claim 1, characterized in that step a) is carried out with an optically pure amine of the formula (III) in which $X_1$ is different from $X_2$.

**3.** Process according to claim 1, characterized in that step a) is carried out with an amine of the formula (III) in which $X_1$ is different from $X_2$, in the racemic form, and in that the mixture of epimers can be resolved after any step of the process or in the final step.

**4.** Process according to one of claims 1 or 3, characterized in that the amine of the formula (III) used in step a) is d,l-α-methyltryptamine and the phenol of the formula (VIII) used in step e) is orthocresol.

**5.** Process according to claim 1, characterized in that the amine of the formula (III) used in step a) is α,α-dimethyltryptamine and the phenol derivative of the formula (VIII) used in step e) is 4-hydroxyindole.

**6.** Process according to any one of the preceding claims, characterized in that the oxycarbonyl group

ROCO involved in step b) is a tert.-butoxycarbonyl or methoxycarbonyl group.

**7.** Process according to any one of claims 1 to 6, characterized in that the heating in step c) is carried out in a water/alcohol mixture and must be controlled.

**8.** Process according to any one of claims 1 to 6, characterized in that the heating in step c) is carried out in a non-aqueous solvent, such as dimethylformamide.

**9.** Process according to any one of the preceding claims, characterized in that the alkaline agent involved in step e) is selected from the group consisting of sodium hydroxide, potassium hydroxide, a sodium or potassium alcoholate and sodium hydride.

**10.** Process according to any one of the preceding claims, characterized in that step f) is carried out in a suitable solvent such as an alcohol, a glycol, a glycol ether or a dipolar solvent such as disulfolan.

**11.** 5-Hydroxymethyl-3-[2-(indol-3-yl)ethyl]-1,3-oxazolidin-2-one derivatives of the formula:

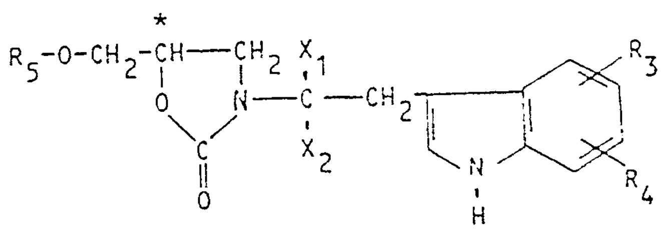

(X)

in which:

- the C* atom has the (R) or (S) configuration;
- $R_5$ represents a hydrogen atom or a group:

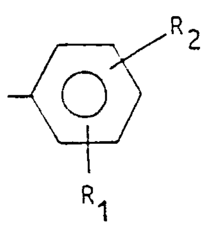

- $X_1$ and $X_2$ each independently denote a hydrogen atom or straight or branched alkyl with 1 to 4 carbon atoms;
- $R_1$ and $R_2$ each independently denote a hydrogen atom, a halogen atom, a straight or branched alkyl with 1 to 4 carbon atoms, a straight or branched alkoxy with 1 to 4 carbon atoms, a formyl, or $R_1$ and $R_2$, taken together with the benzene nucleus to which they are bonded, form a bicyclic nucleus selected form those of formula :

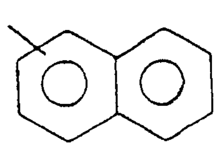 ; 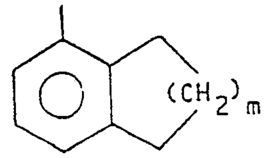 ; 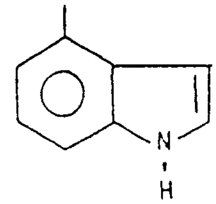

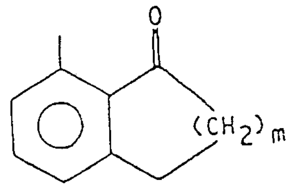 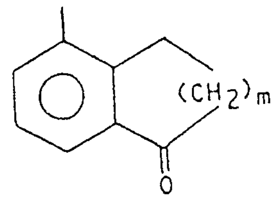

- $R_3$ and $R_4$ each independently denote a hydrogen atom, a halogen atom, a straight or branched alkyl with 1 to 4 carbon atoms or a straight or branched alkoxy with 1 to 4 carbon atoms;
- m represents an integer between 1 and 3.

characterized in that it comprises the steps of

a) condensing 3-tosyloxy-1,2-0-isopropylidenepropane-1,2-diol of the formula (II) in an optically pure form:

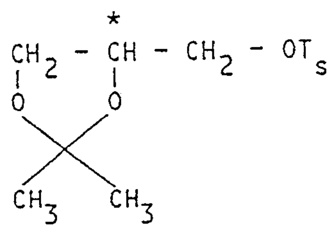

(II)

with a primary amine of the formula (III),

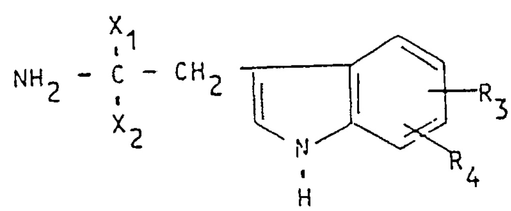

(III)

by heating in an inert solvent, to give the derivative of the formula (IV):

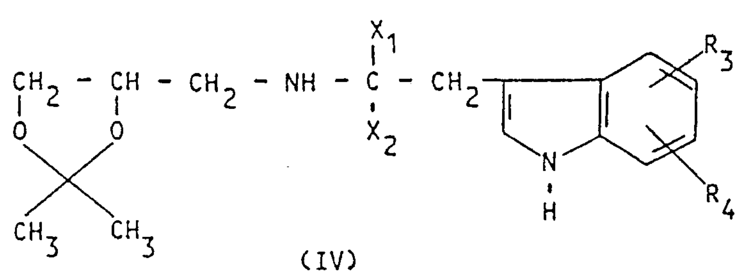

(IV)

b) carrying out in any order the hydrolysis of the dioxolan ring of the compound of formula (IV) in an acid medium and the substitution of the amine functional group of the said compound by an oxycarbonyl group ROCO, in which R represents a lower alkyl group or a benzyl, to form the compound of the formula (VI):

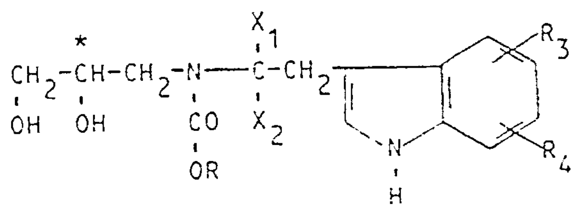

(VI)

c) heating the compound (VI) in a basic medium in order to prepare the oxazolidinone of the formula:

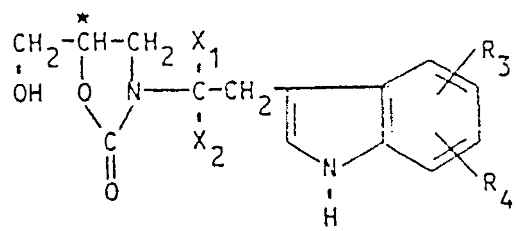

(X)

in which $R_5$ = H;
and in that, the preparation of the compound of formula (X) in which $R_5$ is a group

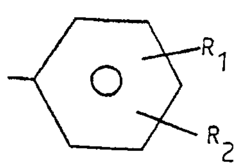

comprises the steps of:
d) esterifying the alcohol functional group of the compound of the formula (X) in which $R_5$ = H, with tosyl chloride or mesyl chloride in pyridine; and
e) reacting the compound esterified in this way, in the presence of an alkaline agent and in an appropriate solvent, with a phenol of formula (VIII)

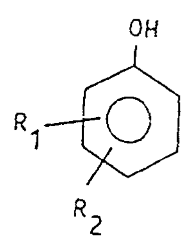

(VIII)

**12.** Process according to claim 11, characterized in that step a) is carried out with an amine of the formula (III), in which $X_1$ is different from $X_2$, the carbon carrying $X_1$ and $X_2$ having the configuration (R) or (S).

**13.** Process according to claim 11, characterized in that step a) is carried out with an amine of the formula (III), in which $X_1$ is different from $X_2$, in the racemic form and in that the derivatives of formula (X) are obtained in the form of mixtures of epimers.

**14.** Process according to claim 11, characterized in that it is used for the preparation of:
- (S)(+)-3-[1,1-dimethyl-2-(indol-3-yl)ethyl]-5-[(indol-4-yl)oxymethyl]-1,3-oxazolidin-2-one;
- (2S,1'R)-3-[2-(indol-3-yl)-1-methylethyl]-5-(2-methylphenoxymethyl)-1,3-oxazolidin-2-one;
- (2S,1'S)-3-[2-(indol-3-yl)-1-methylethyl]-5-(2-methylphenoxymethyl)-1,3-oxazolidin-2-one,

using the adequate reagents of formulae (III) and (VIII).

**Patentansprüche**

**Patentansprüche für folgende Vertragsstaaten : BE, CH, DE, FR, GB, GR, IT, LI, LU, NL, SE**

**1.** Verfahren zur stereospezifischen Synthese von Indolderivaten der Formel I

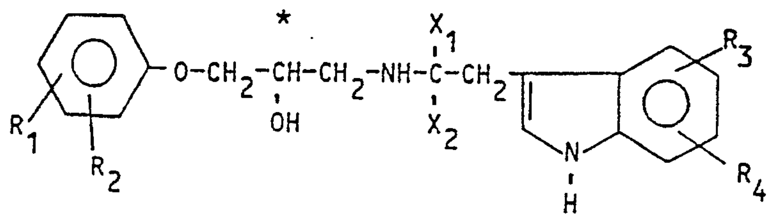

(I),

worin bedeuten:

- C* ein C-Atom mit R- oder S-Konfiguration;
- $X_1$ und $X_2$ jeweils unabhängig ein Wasserstoffatom oder geradkettiges oder verzweigtes $C_{1-4}$-Alkyl;
- $R_1$ und $R_2$ jeweils unabhängig ein Wasserstoffatom, ein Halogenatom, geradkettiges oder verzweigtes $C_{1-4}$-Alkyl, geradkettiges oder verzweigtes $C_{1-4}$-Alkoxy oder Formyl

  oder $R_1$ und $R_2$ zusammen mit, dem Benzolring, an dem sie gebunden sind, ein bicyclisches Ringsystem einer der Formeln

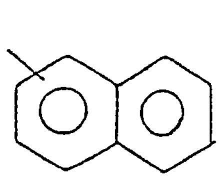 ; 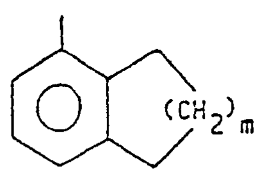 ; 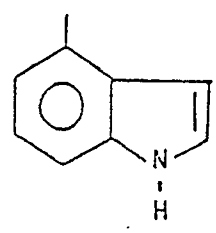

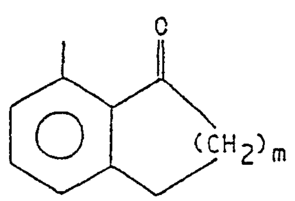 ; 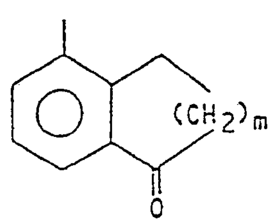 ;

- $R_3$ und $R_4$ jeweils unabhängig ein Wasserstoffatom, ein Halogenatom, geradkettiges oder verzweigtes $C_{1-4}$-Alkyl oder geradkettiges oder verzweigtes $C_{1-4}$-Alkoxy

und

- m eine ganze Zahl von 1 bis 3,

sowie ihrer pharmazeutisch akzeptablen Salze mit organischen oder anorganischen Säuren,

**gekennzeichnet durch :**

a) Kondensation des 3-Tosyloxy-1,2-0-isopropyliden-1,2-propandiols der Formel II

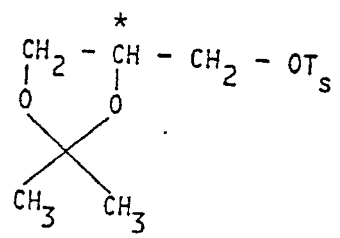

(II)

in optisch reiner Form mit einem primären Amin der Formel III

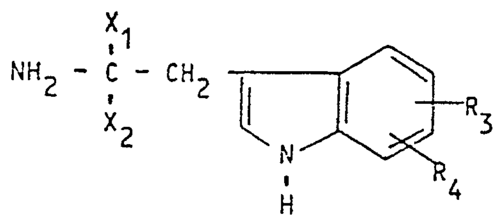

(III)

durch Erhitzen in einem inerten Lösungsmittel unter Erhalt des Derivats der Formel IV

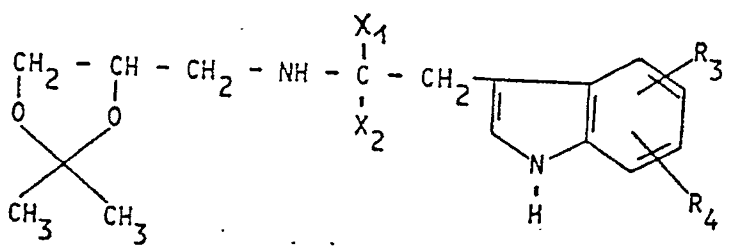

(IV);

b) Hydrolyse des Dioxolanrings der Verbindung der Formel IV in saurem Medium und Substitution der Aminfunktion dieser Verbindung durch eine Oxycarbonylgruppe ROCO, in der R geradkettiges oder verzweigtes $C_{1-4}$- Alkyl oder Benzyl bedeutet,

wobei diese Schritte in beliebiger Reihenfolge durchgeführt werden können,

unter Erhalt der Verbindung der Formel VI

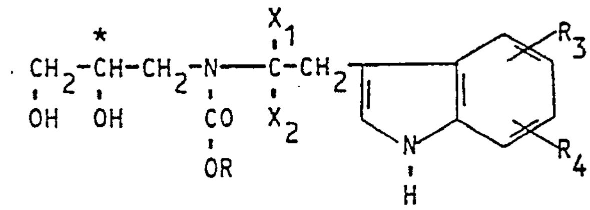

(VI);

c) Erhitzen der Verbindung VI in basischem Medium unter Erhalt des Oxazolidinons der Formel VII

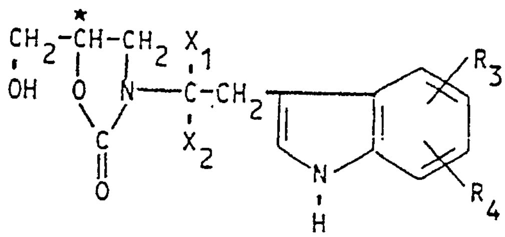

(VII);

d) Veresterung der Alkoholfunktion der Verbindung der Formel VII mit Tosylchlorid oder Mesylchlorid in Pyridin;

e) Umsetzung der so veresterten Verbindung der Formel VII in Gegenwart eines alkalischen Mittels und in einem geeigneten Lösungsmittel mit einem Phenol der Formel VIII

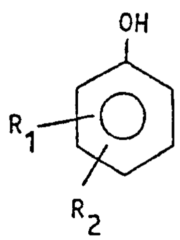

(VIII)

unter Erhalt der Verbindung der Formel IX

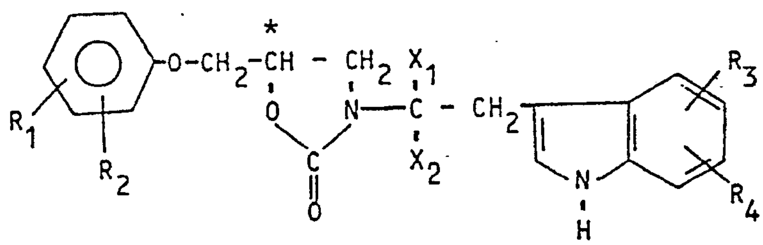

(IX);

f) Erhitzen der Verbindung der Formel IX in einem basischen Medium in einem geeigneten Lösungsmittel in Gegenwart von Wasser am Rückfluß und Isolieren der erhaltenen Verbindung der Formel I

g) und gegebenenfalls Umwandlung der Verbindung der Formel I in ein pharmazeutisch akzeptables Salz durch Einwirkung einer anorganischen oder organischen Säure.

**2.** Verfahren nach Anspruch 1, dadurch gekennzeichnet, daß in Stufe a) ein Amin der Formel III, in der $X_1$ von $X_2$ verschieden ist, in optisch reiner Form eingesetzt wird.

**3.** Verfahren nach Anspruch 1, dadurch gekennzeichnet, daß in Stufe a) ein Amin der Formel III, in der $X_1$

von $X_2$ verschieden ist, in racemischer Form eingesetzt wird und die Trennung des Epimerengemischs nach jeder Stufe des Verfahrens oder in der letzten Stufe vorgenommen werden kann.

**4.** Verfahren nach einem der Ansprüche 1 oder 3, dadurch gekennzeichnet, daß in Stufe a) als Amin der Formel III D,L-α-Methyltryptamin und in Stufe e) als Phenol der Formel VIII o-Cresol eingesetzt werden.

**5.** Verfahren nach Anspruch 1, dadurch gekennzeichnet, daß in Stufe a) als Amin der Formel III α,α-Dimethyltryptamin und in Stufe e) als Phenolderivat der Formel VIII 4-Hydroxyindol eingesetzt werden.

**6.** Verfahren nach einem der vorhergehenden Ansprüche, dadurch gekennzeichnet, daß die Oxycarbonylgruppe ROCO in Stufe b) t-Butoxycarbonyl oder Methoxycarbonyl ist.

**7.** Verfahren nach einem der Ansprüche 1 bis 6, dadurch gekennzeichnet, daß das Erhitzen in Stufe c) in einem Wasser-Alkohol-Gemisch durchgeführt wird, wobei das Erhitzen kontrolliert werden muß.

**8.** Verfahren nach einem der Ansprüche 1 bis 6, dadurch gekennzeichnet, daß das Erhitzen in Stufe c) in einem nichtwäßrigen Lösungsmittel, wie Dimethylformamid, durchgeführt wird.

**9.** Verfahren nach einem der vorhergehenden Ansprüche, dadurch gekennzeichnet, daß das in Stufe e) eingesetzte alkalische Mittel unter Natriumhydroxid, Kaliumhydroxid, einem Natrium- oder Kaliumalkoholat und Natriumhydrid ausgewählt wird.

**10.** Verfahren nach einem der vorhergehenden Ansprüche, dadurch gekennzeichnet, daß Stufe f) in einem geeigneten Lösungsmittel, wie einem Alkohol, einem Glycol oder einem Glycolether, oder einem dipolaren Lösungsmittel, wie Disulfolan, durchgeführt wird.

**11.** Derivate des 5-Hydroxymethyl-3-[2-(3-indolyl)-ethyl]-1,3-oxazolidin-2-ons der Formel X,

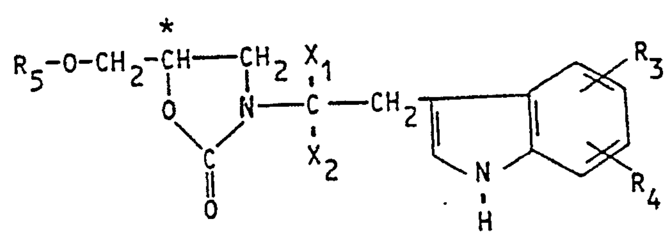

(X),

in der bedeuten:

- $C^*$ ein C-Atom mit R- oder S-Konfiguration;
- $R_5$ ein Wasserstoffatom oder eine Gruppe

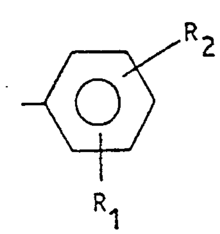

;

- $X_1$ und $X_2$ jeweils unabhängig ein Wasserstoffatom oder geradkettiges oder verzweigtes $C_{1-4}$-Alkyl;
- $R_1$ und $R_2$ jeweils unabhängig ein Wasserstoffatom, e in Halogenatom, geradkettiges oder verzweigtes $C_{1-4}$- Alkyl, geradkettiges oder verzweigtes $C_{1-4}$-Alkoxy oder Formyl

oder $R_1$ und $R_2$ zusammen mit dem Benzolring, an dem sie gebunden sind, ein bicyclisches Ringsystem einer der Formeln

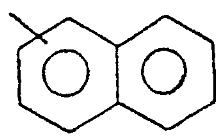 ; 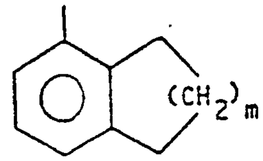 ; 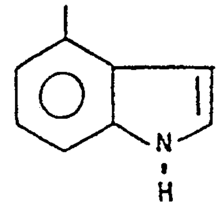

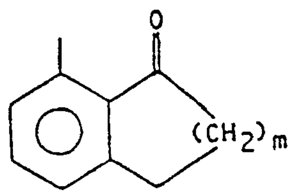 ; 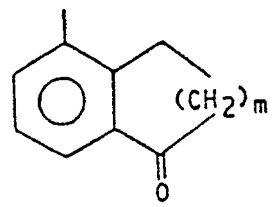 ;

- $R_3$ und $R_4$ jeweils unabhängig ein Wasserstoffatom, ein Halogenatom, geradkettiges oder verzweigtes $C_{1-4}$- Alkyl oder geradkettiges oder verzweigtes $C_{1-4}$- Alkoxy

  und

- m eine ganze Zahl von 1 bis 3.

**12.** Derivate der Formel X nach Anspruch 11, in der $X_1$ von $X_2$ verschieden ist, wobei das die Substituenten $X_1$ und $X_2$ tragende Kohlenstoffatom R- oder S-Konfiguration aufweist.

**13.** Derivate der Formel X nach Anspruch 11, in der $X_1$ von $X_2$ verschieden ist, wobei die Derivate als Epimerengemische vorliegen.

**14.** Derivate nach Anspruch 11, die ausgewählt sind unter:

(S)-(+)-3-[1,1-Dimethyl-2-(3-Indolyl)-ethyl] - [5-(4-indolyloxymethyl)] - 1,3-oxazolidin-2-on;

(2S,1'R)-3-[2-(3-Indolyl)-1-methylethyl]-5-(2-methylphenoxymethyl)-1,3-oxazolidin-2-on;

(2S,1'S)-3-[2-(3-Indolyl)-1-methylethyl]-5-(2-methylphenoxymethyl)-1,3-oxazolidin-2-on.

**Patentansprüche für folgende Vertragsstaaten : AT, ES**

**1.** Verfahren zur stereospezifischen Synthese von Indolderivaten der Formel I,

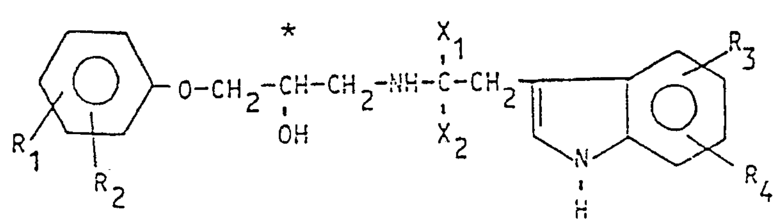 (I),

worin bedeuten:

- C* ein C-Atom mit R- oder S-Konfiguration;
- $X_1$ und $X_2$ jeweils unabhängig ein Wasserstoffatom oder geradkettiges oder verzweigtes $C_{1-4}$-Alkyl;
- $R_1$ und $R_2$ jeweils unabhängig ein Wasserstoffatom, ein Halogenatom, geradkettiges oder verzweigtes $C_{1-4}$-Alkyl, geradkettiges oder verzweigtes $C_{1-4}$-Alkoxy oder Formyl

  oder $R_1$ und $R_2$ zusammen mit dem Benzolring, an dem sie gebunden sind, ein bicyclisches Ringsystem einer der Formeln

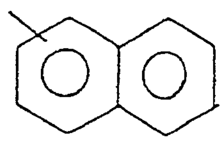 ; 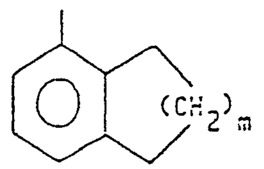 ; 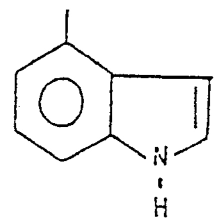

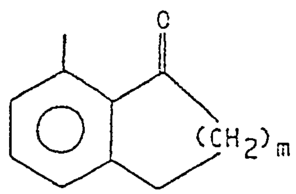 ; 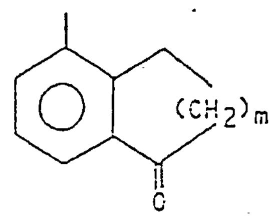 ;

- $R_3$ und $R_4$ jeweils unabhängig ein Wasserstoffatom, ein Halogenatom, geradkettiges oder verzweigtes $C_{1-4}$-Alkyl oder geradkettiges oder verzweigtes $C_{1-4}$-Alkoxy

  und
- m eine ganze Zahl von 1 bis 3,

sowie ihrer pharmazeutisch akzeptablen Salze mit organischen oder anorganischen Säuren,

**gekennzeichnet durch :**

a) Kondensation des 3-Tosyloxy-1,2-0-isopropyliden-1,2-propandiols der Formel II

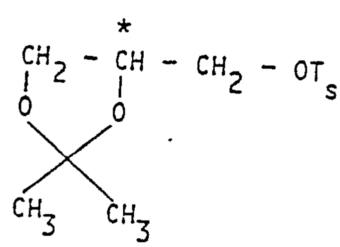

(II)

in optisch reiner Form mit einem primären Amin der Formel III

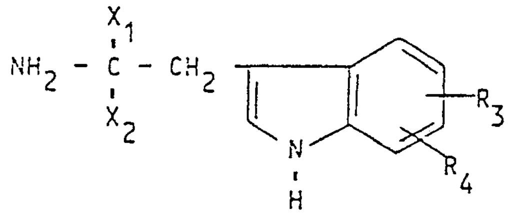

(III)

durch Erhitzen in einem inerten Lösungsmittel unter Erhalt des Derivats der Formel IV

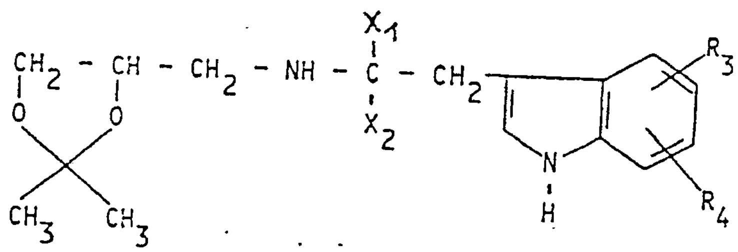

(IV);

b) Hydrolyse des Dioxolanrings der Verbindung der Formel IV in saurem Medium und Substitution der Aminfunktion dieser Verbindung durch eine Oxycarbonylgruppe ROCO, in der R geradkettiges oder verzweigtes $C_{1-4}$-Alkyl oder Benzyl bedeutet,

wobei diese Schritte in beliebiger Reihenfolge durchgeführt werden können,

unter Erhalt der Verbindung der Formel VI

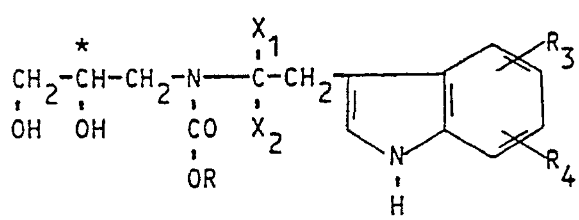

(VI);

c) Erhitzen der Verbindung VI in basischem Medium unter Erhalt des Oxazolidinons der Formel VII

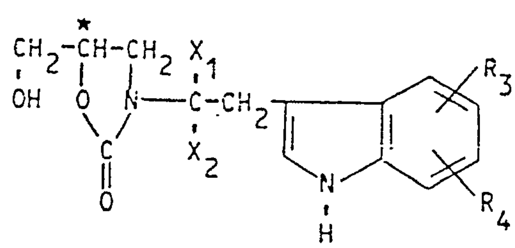

(VII);

d) Veresterung der Alkoholfunktion der Verbindung der Formel VII mit Tosylchlorid oder Mesylchlorid in Pyridin;
e) Umsetzung der so veresterten Verbindung der Formel VII in Gegenwart eines alkalischen Mittels und in einem geeigneten Lösungsmittel mit einem Phenol der Formel VIII

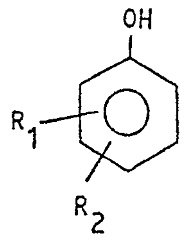

(VIII)

unter Erhalt der Verbindung der Formel IX

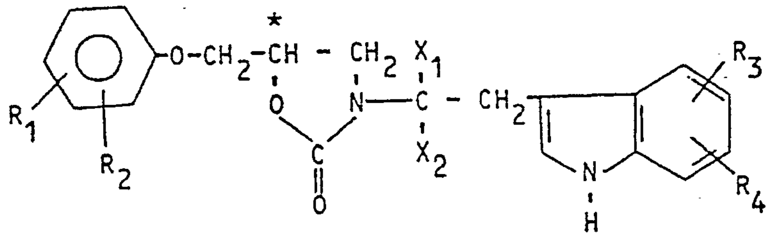

(IX);

f) Erhitzen der Verbindung der Formel IX in einem basischen Medium in einem geeigneten Lösungsmittel in Gegenwart von Wasser am Rückfluß und Isolieren der erhaltenen Verbindung der Formel I

g) und gegebenenfalls Umwandlung der Verbindung der Formel I in ein pharmazeutisch akzeptables Salz durch Einwirkung einer anorganischen oder organischen Säure.

**2.** Verfahren nach Anspruch 1, dadurch gekennzeichnet, daß in Stufe a) ein Amin der Formel III, in der $X_1$ von $X_2$ verschieden ist, in optisch reiner Form eingesetzt wird.

**3.** Verfahren nach Anspruch 1, dadurch gekennzeichnet, daß in Stufe a) ein Amin der Formel III, in der $X_1$ von $X_2$ verschieden ist, in racemischer Form eingesetzt wird und die Trennung des Epimerengemischs nach jeder Stufe des Verfahrens oder in der letzten Stufe vorgenommen werden kann.

**4.** Verfahren nach einem der Ansprüche 1 oder 3, dadurch gekennzeichnet, daß in Stufe a) als Amin der Formel III D,L-$\alpha$-Methyltryptamin und in Stufe e) als Phenol der Formel VIII o-Cresol eingesetzt werden.

**5.** Verfahren nach Anspruch 1, dadurch gekennzeichnet, daß in Stufe a) als Amin der Formel III $\alpha$,$\alpha$-Dimethyltryptamin und in Stufe e) als Phenolderivat der Formel VIII 4-Hydroxyindol eingesetzt werden.

**6.** Verfahren nach einem der vorhergehenden Ansprüche, dadurch gekennzeichnet, daß die Oxycarbonylgruppe ROCO in Stufe b) t-Butoxycarbonyl oder Methoxycarbonyl ist.

**7.** Verfahren nach einem der Ansprüche 1 bis 6, dadurch gekennzeichnet, daß das Erhitzen in Stufe c) in einem Wasser-Alkohol-Gemisch durchgeführt wird; wobei das Erhitzen kontrolliert werden muß.

**8.** Verfahren nach einem der Ansprüche 1 bis 6, dadurch gekennzeichnet, daß das Erhitzen in Stufe c) in einem nichtwäßrigen Lösungsmittel, wie Dimethylformamid, durchgeführt wird.

**9.** Verfahren nach einem der vorhergehenden Ansprüche, dadurch gekennzeichnet, daß das in Stufe e) eingesetzte alkalische Mittel unter Natriumhydroxid, Kaliumhydroxid, einem Natrium- oder Kaliumalkoholat und Natriumhydrid ausgewählt wird.

**10.** Verfahren nach einem der vorhergehenden Ansprüche, dadurch gekennzeichnet, daß Stufe f) in einem geeigneten Lösungsmittel, wie einem Alkohol, einem Glycol oder einem Glycolether, oder einem dipolaren Lösungsmittel, wie Disulfolan, durchgeführt wird.

**11.** Verfahren zur Herstellung von Derivaten des 5-Hydroxy-methyl-3-[2-(3-indolyl)-ethyl]-1,3-oxazolidin-2-ons der Formel X,

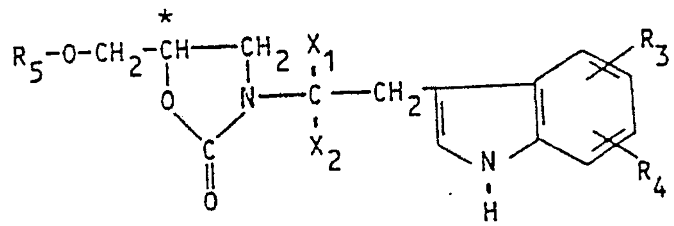

(X),

in der bedeuten:

- C* ein C-Atom mit R- oder S-Konfiguration;
- $R_5$ ein Wasserstoffatom oder eine Gruppe

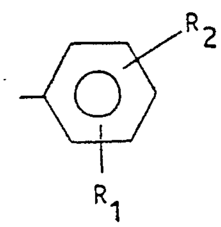

;

- $X_1$ und $X_2$ jeweils unabhängig ein Wasserstoffatom oder geradkettiges oder verzweigtes $C_{1-4}$-Alkyl;
- $R_1$ und $R_2$ jeweils unabhängig ein Wasserstoffatom, ein Halogenatom, geradkettiges oder verzweigtes $C_{1-4}$- Alkyl, geradkettiges oder verzweigtes $C_{1-4}$-Alkoxy oder Formyl

  oder $R_1$ und $R_2$ zusammen mit dem Benzolring, an dem sie gebunden sind, ein bicyclisches Ringsystem einer der Formeln

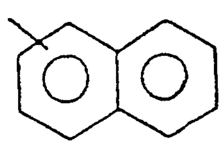

;

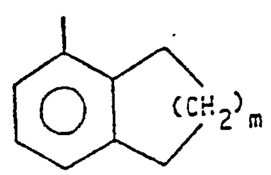

;

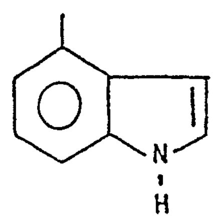

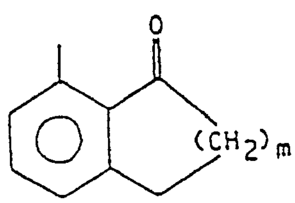

;

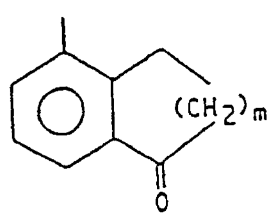

;

- $R_3$ und $R_4$ jeweils unabhängig ein Wasserstoffatom, ein Halogenatom, geradkettiges oder verzweigtes $C_{1-4}$- Alkyl oder geradkettiges oder verzweigtes $C_{1-4}$- Alkoxy

und

- m eine ganze Zahl von 1 bis 3,

**gekennzeichnet durch :**

a) Kondensation des 3-Tosyloxy-1,2-0-isopropyliden-1,2-propandiols der Formel II

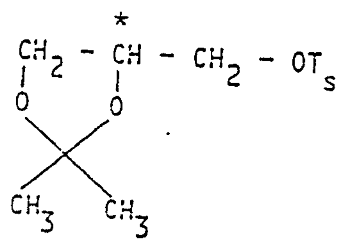

(II),

in optisch reiner Form mit einem primären Amin der Formel III

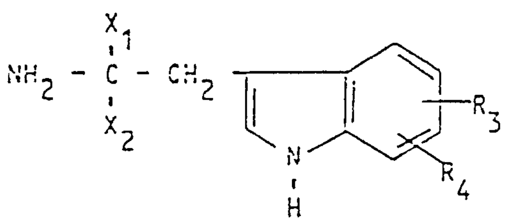

(III)

durch Erhitzen in einem inerten Lösungsmittel unter Erhalt des Derivats der Formel IV

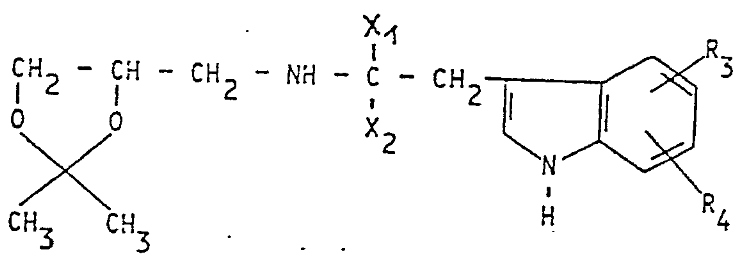

(IV);

b) Hydrolyse des Dioxolanrings der Verbindung der Formel IV in saurem Medium und Substitution der Aminfunktion dieser Verbindung durch eine Oxycarbonylgruppe ROCO, in der R geradkettiges oder verzweigtes $C_{1-4}$-Alkyl oder Benzyl bedeutet,

wobei diese Schritte in beliebiger Reihenfolge durchgeführt werden können,

unter Erhalt der Verbindung der Formel VI

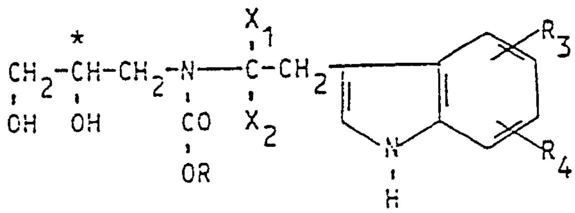 (VI);

c) Erhitzen der Verbindung VI in einem basischen Medium unter Erhalt des Oxazolidinons der Formel X

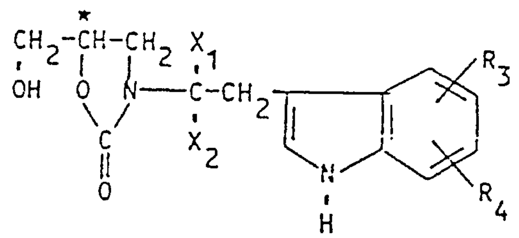 (X)

mit $R_5$ = H

sowie zur Herstellung der Verbindung der Formel X, in der $R_5$ eine Gruppe

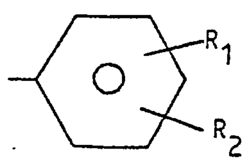

bedeutet,
d) Veresterung der Alkoholfunktion der Verbindung der Formel X mit $R_5$ = H mit Tosylchlorid oder Mesylchlorid in Pyridin

und

e) Umsetzung der so veresterten Verbindung in Gegenwart eines alkalischen Mittels und in einem geeigneten Lösungsmittel mit einem Phenol der Formel VIII

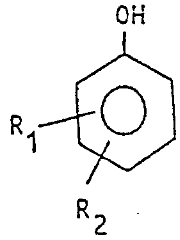 (VIII).

**12.** Verfahren nach Anspruch 11, dadurch gekennzeichnet, daß in Schritt a) ein Amin der Formel III eingesetzt wird, in der $X_1$ von $X_2$ verschieden ist, wobei das die Substituenten $X_1$ und $X_2$ tragende

Kohlenstoffatom R- oder S-Konfiguration besitzt.

**13.** Verfahren nach Anspruch 11, dadurch gekennzeichnet, daß in Schritt a) ein Amin der Formel III, in oder $X_1$ von $X_2$ verschieden ist, in racemischer Form eingesetzt wird und die Derivate der Formel X in Form von Epimerengemischen erhalten werden.

**14.** Verfahren nach Anspruch 11, gekennzeichnet durch Herstellung folgender Verbindungen:

(S)-( + )-3-(1,1-Dimethyl-3-indolylethyl)-5-(4-indolyloxymethyl)-1,3-oxazolidin-2-on;

(2S,1'R)-3-[2-(3-Indolyl)-1-methylethyl]-5-(2-methylphenoxymethyl)-1,3-oxazolidin-2-on;

(2S,1'S)-3-[2-(3-Indolyl)-1-methylethyl]-5-(2-methylphenoxymethyl)-1,3-oxazolidin-2-on

unter Verwendung geeigneter Reaktanten der Formeln III und VIII.